# EUROPEAN PATENT APPLICATION

(11) **EP 2 589 661 A1**
(43) Date of publication of application: **08.05.2013**
(21) Application number: 11187792.4
(22) Date of filing: 04.11.2011
(51) Int. Cl.: C12N 15/85, C12N 15/67

(54) **Posttranscriptional Element**

(71) Applicant: Veterinärmedizinische Universität Wien, 1210 Vienna (AT)
(72) Inventor: Indik, Stanislav, 1210 Vienna (AT)
(74) Representative: Sonn & Partner Patentanwälte

(57) **Abstract**

The invention discloses the use of the 5' terminal region of the env mRNA of Mouse Mammary Tumour Virus (MMTV) for enhancing nucleo-cytoplasmic translocation of a heterologous gene expressed in a host cell.

## Description

The invention relates to expression vectors and methods for expressing heterologous genes with such vectors in eukaryotic hosts.

Expression systems in eukaryotic hosts require efficient means for transcription of the gene to be expressed, for transport of the mRNA into the cytoplasm, suitable elements that allow proper transcription of the gene into the protein and often appropriate post-translatorial equipment, including transportation signals.

Model systems to study gene expression often use viruses and viral expression elements. Viruses require efficient expression of their genes to produce infectious progeny. Therefore, they have developed several strategies to control gene expression in a competitive cellular environment. It has become apparent that a regulation of gene expression at the posttranscriptional level, including the control of splicing, mRNA transcript stability, nucleo-cytoplasmic export, sub-cellular localization and translation, is widespread among viruses. Retroviral RNAs contain several highly structured motifs that modulate various posttranscriptional steps in the expression of viral RNAs. Complex retroviruses such as the prototypic lentivirus human immunodeficiency virus type 1 (HIV-1) and complex betaretroviruses including mouse mammary tumour virus (MMTV), human endogenous retrovirus K (HERV-K) and Jaagsiekte sheep retrovirus (JSRV) carry a cis-acting responsive element that is recognized by virus-encoded Rev-like proteins. The protein/RNA interaction facilitates an efficient nucleo-cytoplasmic transport of the unspliced (single spliced) viral transcripts via the CRM1/exportin 1 pathway. Simple retroviruses such as Mason-Pfizer monkey virus (MPMV) and simian retrovirus (SRV) lack analogous Rev-like regulatory proteins. Instead, they directly recruit the cellular nuclear RNA export factor 1 (NXF1/Tap) to the structured cis-acting constitutive transport element (CTE) positioned near the 3' end of viral RNAs. This interaction escorts the nascent viral RNA out of the nucleus and enhances RNA stability. Rous sarcoma virus (RSV) is another simple retrovirus that requires a cis-acting RNA element for nuclear export. The direct repeats (DR) of RSV are responsible for the accumulation of viral RNA in the cytoplasm.

The 5' leader sequence of retroviral RNA is also known to contain extensively structured motifs. Experiments with spleen necrosis virus (SNV) demonstrated that this region has a pronounced positive effect on virus gene expression. The presence of the SNV 5' leader in an HIV-1-Gag expression reporter construct facilitated Rev-responsive element (RRE) / Rev-independent expression of the HIV-1 Gag protein. Thus, the RU5 fragment of the SNV LTR was sufficient to overcome the cis-acting inhibitory sequences (INSs) located in the HIV Gag-encoding mRNA which are responsible for low steady-state cytoplasmic levels of this mRNA in the absence of Rev. Interestingly, similar sequences able to de-repress INSs were also found in other retroviruses, including reticuloendotheliosis virus A (REV-A), human T-cell leukemia virus type 1 (HTLV-1), Mason-Pfizer monkey virus (MPMV), feline leukemia virus (FeLV), human foamy virus (HFV), and bovine leukemia virus (BLV). Further experiments attempting to pinpoint the molecular mechanism of the augmented expression revealed that the 5' leader functions as a positive posttranscriptional control element (PCE) that positively affects translational utilization of cytoplasmic mRNAs. Although the steady-state cytoplasmic mRNA levels remain virtually unchanged in the presence of the 5' leader, a markedly greater protein production is observed.

A betaretrovirus closely related to MMTV, JSRV, appears to utilize another mechanism to ensure a high level of gene expression. Recently, evidence was provided demonstrating that the presence of the 5' leader together with the 3'LTR in an Env expression construct markedly increased Env production. A combination of positive effects on the env mRNA export from the nucleus, enhanced mRNA stability and reduced intra-env splicing was shown to be responsible for this phenomenon. Interestingly, although JSRV encodes an orthologue of HIV-1 Rev, Rej, nucleocyto-plasmic translocation of the env mRNA seems to be achieved independently of this auxiliary protein as treatment of cells with an inhibitor of the Crm1 (leptomycin B) did not decrease Env expression. Therefore, it appears that the JSRV env mRNA is, in contrast to the env mRNA of other complex retroviruses exported from the nucleus via a Crm1-independent pathway.

A Rev-like protein Rem, encoded by MMTV is essential for cytoplasmic accumulation of the unspliced viral RNA (Mertz et al., J. Virol. 79 (2005), 14737-14747). However, by analogy to JSRV, Rem appears to be dispensable for nuclear export of the env mRNA. Leptomycin B treatment as well as deletion of the Rem responsive element did not decrease cytoplasmic env mRNA levels.

On eukaryotic level, yeast and metazoan cells can serve as model systems to study nuclear RNA export pathways. Bulk mRNA is exported from the nucleus by the non-karyopherin type receptor, Tap. In contrast to the CTE-bearing RNAs, cellular mRNAs do not associate with Tap directly. Instead, this interaction is mediated by various adaptor proteins. Genetic screens in yeasts significantly contributed to the identification of these factors. In yeast, Yra1p (Aly in metazoans) has been identified as a Mex67p (Tap in metazoans) binding partner crucial for mRNA export. Yra1p is recruited to mRNA transcripts via interaction with the splicing factor Sub2p (UAP56 in metazoans). Yra1p and Sup2p are members of the conserved multiprotein TREX (transcription/export) complex (comprised in metazoans of Aly, UAP56 and the Tho complex) that is recruited co-transcriptionally to mRNA in yeast and to the 5' end of mRNA during splicing in metazoans. Current models favor a situation where a DEAD-box helicase UAP56, which is recruited to the metazoan spliceosomes, functions as a bridge linking splicing and mRNA export. UAP56 recruits other cellular proteins required for nuclear export, such as Aly and the Tho complex. Once assembled, the TREX complex associates with the heterodimer Tap/p15, which interacts with the components of nuclear pore complex and mediates mRNA export. This model might explain the observation that spliced mRNAs are exported from the nucleus more efficiently than intronless versions of the same mRNAs. Furthermore, the TREX complex is often recruited to the first exon near the 5'end of an mRNA. This is achieved via an interaction between the TREX component Aly and a cap-binding protein CBP80, ensuring mRNA export to the cytoplasm in a 5' to 3' direction.

Recent results suggest that the metazoan TREX complex is not the only factor required for the nuclear export of bulk mRNAs. Results demonstrated that, in contrast to Yra1p in yeast, Aly is not required for the nuclear export of bulk mRNAs in metazoans. In fact, less than 20% of the *Drosophila melanogaster* (DM) transcriptome was regulated by the TREX complex. Therefore, it appears that the increase in complexity of higher eukaryotic genomes compared to that of yeast is likely to have resulted in a concurrent increase in mRNA network complexity with multiple physiologically relevant adaptors for mRNA export in operation. Consistent with this model a number of adaptor proteins mediating interaction between mRNAs and Tap/p15 heterodimers have been identified (see below). Thus, Tap/p15 is believed to stand at the final step of mRNA export pathways for most mRNAs hence integrating several mRNA export pathways.

A subset of mammalian splicing factors, SR (serine/arginine-rich) proteins, such as 9G8 and SRp20, has been shown to function as adaptor proteins to recruit Tap onto mRNA. These proteins, which shuttle continuously between the nucleus and cytoplasm, bind to exon sequences in pre-mRNAs and recruit spliceosomes to the flanking 5' and 3' splice sites. After splicing, SR proteins remain associated with mRNA, mediate mRNA export from the nucleus and stimulate translation of mRNAs in the cytoplasm. Interestingly, the interaction of SR proteins with the export receptor Tap is regulated by cell cycle-dependent phosphorylation-dephosphorylation events, suggesting an intriguing possibility of cell growth-specific regulation of mRNAs export.

RNA-binding motif protein 15 (RBM15) has been shown to directly bind to the RTE and promote RNA export from the nucleus. RMB15 also specifically binds to Tap, thereby linking the RTE-containing RNA to the Tap export pathway.

Recent work identified a RNA helicase closely related to UAP56, URH49, as a novel adaptor protein mediating mRNA export. Comparison of transcripts affected by downregulation of URH49 revealed that they substantially differ from those affected by depletion of UAP56. Therefore, it was proposed that URH49, associated with CIP29, forms a novel mRNA export complex the AREX (alternative mRNA export) complex, regulating export of a subset of mRNAs different from those mediated by the TREX complex.

A metazoan-specific component of the TREX complex, Thoc5, has also been identified as a Tap interacting partner mediating nuclear export of a subset of mRNAs. RNAi-mediated depletion of Thoc5 resulted in the nuclear accumulation of heat-shock protein 70 (HSP70) mRNA. A similar effect has been observed when Aly and Tap expression was downregulated, suggesting that Thoc5 functions as a co-adaptor for the nuclear export of the HSP70 mRNA.

A recent genome-wide analysis of mRNA export requirements in *Drosophila melanogaster* cells confirmed that the TREX complex regulates only a subset of mRNAs and identified 72 factors required for the export of bulk mRNAs from the nucleus, 52 of which have not been previously implicated in mRNA export pathways. Furthermore, evidence was provided showing specificity in the export requirements of different transcripts. In particular, differential export requirements for unspliced (HSP70) and spliced (HSP83) transcripts have been described. These results further substantiated the commonly held belief that the export pathways of all mRNAs are not identical and that there are specific mRNA export pathways for certain classes of mRNAs, which may be related to the different functions of the final translated products.

One such specialized class of mRNAs encodes for secretory proteins. These mRNAs are often translated by ribosomes, which are targeted to the endoplasmic reticulum (ER) membrane by hydrophobic signal sequences of nascent polypeptides. Previously, it has been proposed that these mRNAs may also associate with the ER membrane in a ribosome-independent manner by interacting with mRNA binding proteins. Recent data suggest that there is a specific export pathway for mRNAs encoding secretory proteins in mammalian cells. It was shown that the signal sequence coding region (SSCR) derived from mouse major histocompatibility complex class 2 molecule H2-K, as well as from human insulin is able to drive nucleo-cytoplasmic translocation of mRNAs and directs the exported mRNAs to the ER membrane. The same transcript lacking the SSCR remained in the nucleus and the export defect could be restored by adding either an intron or SSCR into the pre-mRNAs. The SSCR-mediated nuclear export pathway requires the core export receptor Tap, but is likely independent of the TREX complex. The mechanism by which SSCRs are recognized has not been extensively investigated. However, a strong bias was noticed against adenine-containing codons within vertebrate SSCRs. Mutation of adenine residues within the H2-K SSCR, without altering the amino acid sequence, severely affected the SSCR export signal. Thus, it appears that SSCRs interact with an adaptor protein specifically recognizing adenine-depleted mRNAs, or alternatively that SSCRs fold into a conformation that recruits an export adaptor.

This shows that also for the step of mRNA transport (after transcription) from the nucleus into the cytoplasm (for translation), highly complex mechanisms are present in eukaryotic cells which need to be addressed when gene expression has to be optimized. There is a need for improved expression systems which allow efficient transport of a transcribed gene into the cytoplasm, especially for genes with low expression numbers or genes which are difficult to express. Specifically for DNA vaccines and for gene therapy vectors, low expression levels are usually detrimental for their efficiency. Low transcriptional activity (which includes low transport efficiency out of the nucleus) and low titers of retroviral vectors are currently identified as major barriers to successful gene therapy (Kay, Nat. Rev. Genetics 12 (2011), 316-328) and cell-based therapies (Naldini, Nat. Rev. Genetics 12 (2011), 301-315).

Enhancing overall expression performance is therefore specifically needed in these fields. It was previously suggested to combine two of two retroviral/retroelement-derived cis-acting RNA export elements, RTE and CTE, which synergistically increased expression of different retroviral mRNAs that are otherwise poorly expressed, into expression vectors to provide a tool to improve expression of poorly expressed, unstable retroviral (lentiviral) mRNAs to levels otherwise only achieved via more cumbersome RNA optimization (Smulevitch et al., Retrovirology 3 (2006), 6). However, there are obviously many parameters relevant for efficient expression of recombinant genes in given host cells, especially for the effective transport of the mRNA for such genes out of the nucleus into the cytoplasm.

It is therefore an object of the present invention to provide improved means for eukaryotic expression systems, specifically for enhancing the nucleo-cytoplasmic transport of the transcribed mRNA of a gene being of heterologous nature for the host cell.

Therefore, the invention provides the use of the 5' terminal region of the env mRNA of Mouse Mammary Tumour Virus (MMTV) for enhancing nucleo-cytoplasmic translocation of a heterologous gene expressed in a host cell.

Within the course of the present invention it was found that the 5' leader of MMTV (also referred to as "MMTV-derived positive posttranscriptional element" (MPPE)) has an effect on gene expression. Using a series of MMTV Env expression constructs and firefly luciferase gene reporter plasmids, a fragment within the 5' leader located downstream of the R and U5 regions was identified that augmented expression of the MMTV env and firefly luciferase genes. Immunoblot and Northern blot analyses revealed that this region does not exert a PCE-like translational enhancement activity. Rather, the presence of this region increases the cytoplasmic steady-state mRNA levels.

It seems that MMTV employs two distinct RNA elements to export its transcripts out of the nucleus. While transport of the unspliced gag/pol mRNA is mediated by RmRE, which interacts with the virus-encoded accessory protein Rem and subsequently with Crm1 and the components of the nuclear pore complex, spliced mRNAs are likely exported via a Crm1-independent manner. Thus, it appears that MMTV is the first retrovirus for which two distinct RNA export elements have been identified and characterized. The present data show that a cis-acting element (MPPE), positioned near the 5' end of subgenomic transcripts, which is formed during splicing (5' UTR/env junction), drives mRNA transport to this pathway. This element acts as a functional analogue of SSRC and as such affects intracytoplasmic localization of RNA. Therefore, MPPE represents the first example of a cis-acting RNA export element formed during splicing by joining two exon sequences.

MMTV LTR has been used extensively in the prior art in various expression vectors, mainly due to its promoter function, but also to other regulatory elements (see e.g. US 5,814,493 A or WO 2010/056816 A2). MPPE according to the present invention is, however, significantly downstream of MMTV-LTR. Moreover, the MPEE according to the present invention also has parts of env included, presence of which is not necessary if the promoting activity of MMTV LTR is intended to be used. The MMTV promoter, including enhancer, is located at positions 1-1321, that is 1470-150 bp upstream of MPPE.

The primary transcripts of many eukaryotic genes are shortened by removing the intron sequences from the pre-mRNA during splicing. In addition, pre-mRNAs can often be alternatively spliced producing different mRNAs, which then give rise to different proteins (cytoplasmic, membrane-bound, secretory). Therefore, it is conceivable that some of the splicing events lead to the formation of an analogous cis-acting RNA transport element mediating cellular RNA export and localization (e.g. secretory proteins). Thus, MMTV may also be used as a model organism to characterize this RNA export pathway to further aid the understanding of mechanisms involved in cellular mRNA export and localization.

Identification of the novel RNA export element according to the present invention is also valuable for future translational approaches. RNA export elements can be used to ensure an efficient expression of various genes. MPPE surprisingly also enhances the expression of non-viral genes showing its applicability in e.g. DNA vaccination and gene transfer studies. Most importantly, however, the presence of a potent RNA export signal is crucial for the expression of retroviral mRNAs that are otherwise poorly expressed. Insertion of the MMTV-derived element into retrovirus/lentivirus expression vectors can alleviate the downregulatory effect of the inhibitory sequences; allow efficient expression of structural genes, hence facilitating high titer virus/vector production. Vector titer is one of the key issues of viral gene therapy. An inefficient export and stability of viral RNAs is responsible for inefficient generation of vector particles, therefore a potent RNA export system may be useful for the future retroviral/lentiviral vector production. Especially preferred is to combine one or more export elements (such as MPPE, RmRE, CTE, RRE, RTE) on a single mRNA molecule. Combination of two or more export elements may result in a synergistic effect markedly enhancing mRNA export and protein expression in a similar way as described previously (Smulevitch et al., 2006). Furthermore, combination of distinct elements avoids the presence of adjacent repeated sequences (such as multiple CTEs), which may cause plasmid instability during growth in bacteria or unwanted recombinations during reverse transcription when placed on the transfer vectors. Identification of a novel potent RNA export element, which functions in the absence of a Rev-like protein and can be combined with other cis-acting RNA export elements, allows less cumbersome vector production protocols. These protocols, in contrast to the currently used third generation lentiviral production system, would not require the presence of a separate Rev-like protein expression plasmid.

The MMPE region according to the present invention is mainly defined by the stem loop structure formed by this element (see also: example section below). According to the present invention, any MMTV fragment containing this structural element is suitable for performing the present invention. Preferably, the 5' terminal region of the env mRNA of MMTV comprises nucleotides 1470 to 1608 and 6683 to 6871 of the MMTV reference strain BR6 (EMBL: M15122) (and therefore lacks nts 1609 to 6682). The M15122 sequence is disclosed as SEQ. ID. No. 1; the contiguous sequence of nt 1470 to 1608 and 6683 to 6871 containing the contiguous "gg" sequence of nt 1608 and 6683 is disclosed as SEQ. ID. No. 2. This 328 bp sequence contains the posttranscriptional regulation of gene expression element according to the present invention.

The MPPE sequence to be used according to the present invention therefore preferably consist of a contiguous sequence from nt 1608 to 6683 wherein the "g" of nt 1608 is immediately followed by the "g" of nt 6683 containing the transport element according to the present invention, for example on an expression vector without the need for splicing.

The MPPE according to the present invention therefore preferably consists of the 328 bp of nts 1470 to 1608 and 6683 to 6871 of MMTV directly linked to each other. It is, however, also possible to include additional sequences or sequence variations, especially at the 5' and/or at the 3' end which do not negatively affect the functionality of the expression of the heterologous gene. A preferred embodiment with a shorter sequence extends e.g. from nt 1471 to 1608 and 6683 to 6780.

Some sequence variations not significantly affecting the transport ability are known from different strains of MMTV and are well available for a person skilled in the art (e.g. MMTV AF0333807, MTV AF228550, HeJ AF228551, C3H AF228552, proviral GR102, MMTV NC001503, etc.).

The present invention is specifically suited for the expression of viral genes, for example in virus-based vaccines or gene transfer system (e.g. in in vivo or ex vivo cell based therapies). Specifically retroviral genes have proven to be problematic in the past due to the low titers of retroviral vectors, inefficient administration and subtherapeutic efficiency (Kay, 2011). In a preferred embodiment of the present invention, the heterologous gene is a viral gene, preferably a retroviral gene, especially a gene from the human immunodeficiency virus (HIV). Other preferred retroviruses are selected from the genera al-pharetrovirus (e.g. Avian leukosis virus, Rous sarcoma virus), betaretrovirus (e.g. MMTV), gammaretrovirus (e.g. Murine leukemia virus, especially Moloney Murine Leukemia Virus (MMLV); Feline leukemia virus), deltaretrovirus (e.g. Bovine leukemia virus, Human T-lymphotropic virus I and II), epsilonretrovirus (Walleye dermal sarcoma virus), lentivirus (apart from HIV 1, e.g. Simian, Feline immunodeficiency viruses) and spumavirus (e.g. Simian foamy virus).

The present invention is specifically suited to provide improved DNA-based vaccines. The heterologous gene is therefore preferably a vaccination antigen. The nature of the vaccination antigen is not critical and is mainly dependent on the disease that should be prevented (or cured) with the vaccination. Preferred antigens are those for which currently already a defined proteinaceous vaccine exists, i.e. a vaccine with defined antigen structure (e.g. recombinantly produced). For example, preferred antigens to be expressed by use of the present invention are antigens of pathogens eliciting lower respiratory infections, especially viral pathogens, such as influenza A or B, rhinovirus, but also antigens of non-viral pathogens, such as H. influenzae, M. catarrhalis, S. pneumoniae and atypical pathogens such as C. pneumoniae, M. pneumoniae and L. pneumophila; HIV/AIDS, antigens of pathogens causing diarrheal diseases, including the bacterial pathogens Salmonella, Shigella, Staphylococcus, Campylobacter jejuni, Clostridium, Escherichia coli, Yersinia, Vibrio cholerae, and the viral pathogens rotavirus, norovirus, adenovirus and astrovirus; M. tuberculosis antigens, Plasmodium antigens, specifically from Plasmodium falciparum, Plasmodium vivax, Plasmodium knowlesi, Plasmodium ovale curtisi, Plasmodium ovale wallikeri and Plasmodium malariae; a Measles virus antigen, an antigen of Clostridium tetani; an antigen of Treponema pallidum; a hepatitis virus antigen, especially hepatitis A, hepatitis B or hepatitis; an antigen of Trypanosoma cruzi, an antigen of a Flavivirus, e.g. a Dengue virus antigen, a Yellow fever virus antigen, a Japanese encephalitis virus antigen, a tic-borne encephalitis antigen or a West Nile virus antigen; an antigen of Trypanosoma brucei gambiense or Trypanosoma brucei rhodesiense; a Pox virus antigen, an antigen of Leishmania, an antigen of Mycobacterium leprae; a nematode antigen; or an antigen of Onchocerca volvulus.

The present invention can also be applied in the field of tumour vaccines (wherein the heterologous gene is a tumour antigen (e.g. of solid tumours or of leukemias)) or in the field of autoimmune disease treatment (wherein the heterologous gene is an autoimmune antigen; e.g. for desensibilization).

Preferably, the heterologous gene is part of a DNA vaccine or part of a gene therapy vector. The heterologous gene and the MPPE according to the present invention can be provided in established and known vector and vaccine formats in the present field; details thereof are well available to the person skilled in the present art. The vaccines and gene-therapy vectors therefore comprise the features necessary or proposed to be necessary for function without having to be explicitly mentioned here (see e.g. Norley et al. ("Molecular Vaccines and Correlates of Protection") and Mühlebach et al. ("Gammaretroviral and Lentiviral Vectors for Gene Delivery") in Retroviruses; Molecular Biology, Genomics and Pathogenesis; edited by R.Kurth and N. Bannert; Caister Academic Press; pages 309-345 and 347-370, respectively).

The present invention is also specifically usable in the industrial production of recombinant proteins. Suitable host cells for this aspect of the present invention are therefore those eukaryotic cell lines which are currently used or suggested to be used in the production of recombinant protein, preferably a CHO cell, a BHK cell, a Vero cell or a HEK-293 cell, especially a HEK-293T cell. Preferred other host cells also include a cell of a non-human animal, preferably a fish, a bird or a mammal, especially a cat, a dog, a mouse, a hamster, a rat, a cow, a horse, a guinea pig, a goat, a rabbit, a sheep, a macaque or a chimpanzee; or a human cell. Whereas in vivo use of the present invention is possible, according to one preferred embodiment of the present invention, methods for treatment performed on the human and animal body are formally excluded (and, for example limited to in vitro treatment of such host cells). For other circumstances (e.g. where these formal exclusion does not have to be applied), another preferred embodiment may include the administration of vectors and nucleic acids as defined for the present invention to a (human) patient in need thereof in an effective amount.

The present invention is performed in a preferred embodiment by providing the 5' terminal region of the env mRNA of MMTV and the heterologous gene encoded on an expression vector, especially an expression plasmid or a viral expression vector.

Therefore, the present invention also relates to an expression vector for a gene in a host cell for which the gene is heterologous, comprising the heterologous gene and elements enabling transcription and, optionally, translation of the gene in the host cell, characterised in that the vector further comprises the 5' terminal region of the env mRNA of MMTV.

The expression vector according to the present invention may further comprise all necessary elements for proper functioning of the vector within the host cell, e.g. suitable promoters, termination signals, etc. operably linked to each other.

The position of the 5' terminal region of the env mRNA of MMTV on the expression vector is not critical as long as it results in the presence of this element on the mRNA transcribed (and, of course, leaves the gene or other functional elements intact). A preferred position of the MPPE is downstream the coding region of the gene to be expressed before the polyadenylation signal, however, the exact positioning is not critical and it functions well in other positions too.

In order to further enhance the transport efficiency for the mRNA into the cytoplasm, it is preferred to include one or more additional nucleus export elements in the expression vector, preferably selected from Rem responsive element (RmRE), constitutive transport element (CTE), Rev responsive element (RRE) or RNA transport element (RTE).

The heterologous gene of the expression vector is preferably a viral gene, preferably a retroviral gene, especially a HIV gene, an intronless gene or a spliced cDNA.

According to a preferred embodiment of the present invention, the expression vector contains elements enabling transcription, and/or additional elements, such as a promoter (e.g. a promoter derived from retroviruses, especially the Rous sarcoma virus (RSV) promoter, the spleen focus forming virus (SFFV) promoter), other viruses such as the cytomegalovirus (CMV) promoter, the herpes simplex virus thymidine kinase promoter(HSV TK),the simian virus 40 (SV40) promoter), promoters derived from eukaryotic organisms especially the elongation factor 1a (EF1a) promoter, the phosphoglycerate kinase 1 (PGK1) promoter and tissue-specific promoters, preferably *haematopoeitic-lineage promoters,* especially human ankyrin (ANK-1) promoter), a pA signal, internal ribosomal (IRES), other elements stabilizing RNA, e.g. WPRE-Woodchuck hepatitis virus posttranscriptional regulatory element, and - as described above - other positive posttranscriptional elements such as RRE from HIV-1, CTE from (MPMV or SRV-1, SRV-2), RTE from ((rodent) intracisternal A-particle retro-elements) IAP.

According to another aspect, the present invention relates to a method for expressing a heterologous gene in a host cell comprising introducing an expression vector according to the present invention into a host cell and inducing expression of the heterologous gene encoded by the expression vector.

The present invention also refers to an isolated nucleic acid comprising a gene expressible in a host cell, elements enabling transcription and, optionally, translation of the gene in the host cell, characterised in that the vector further comprises the 5' terminal region of the env mRNA of MMTV. The isolated nucleic acid molecule according to the present invention is characterised by the contiguous "gg" sequence of 1608 and 6683 and having at least 50, preferably at least 100, more preferred at least 150, especially at least 200 bp of contiguous nucleic acid sequence extending 5' and/or 3' from this "gg" sequence (into the direction of nt. 1470 and/or 6871, respectively). Preferably, the nucleic acid (or the expression vector) according to the present invention comprises at least 230 contiguous nucleotides of SEQ. ID. No. 2, preferably at least 300, especially at least all 328. It may also contain further sequences, especially those extending 5' and/or 3' of exon sequence of SEQ. ID. No. 1.

The invention is further illustrated by means of the following examples and the drawing figures, yet without being limited thereto.

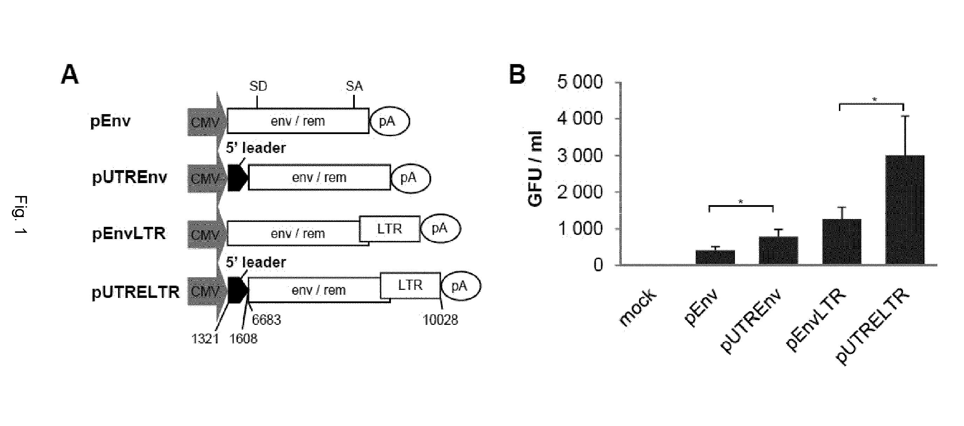

Fig. 1 shows infectivity of MMTV Env-pseudotyped MLV-EGFP virions. (A) Schematic representation of the Env expression constructs used to pseudotype MLV-EGFP particles. All plasmids are based on pcDNA3 (Invitrogen), carrying a CMV IE promoter/enhancer and a bovine growth hormone polyadenylation site (pA). (B) Supernatants from equal number of 2GP19T cells, transiently cotransfected with one of the Env expression constructs and pLXSN-EGFP, were used for infection of CrFK cells. Infectious titers were determined by counting the number of EGFP-expressing cells by FACS. Duplicate infections were performed with equal amounts of supernatant. The data are presented as mean titers (GFU/ml, green fluorescent units per ml) and include standard deviations for each infection. The statistical significance of differences between groups was tested using an unpaired two-tailed Student's t-test. * P<0.05.

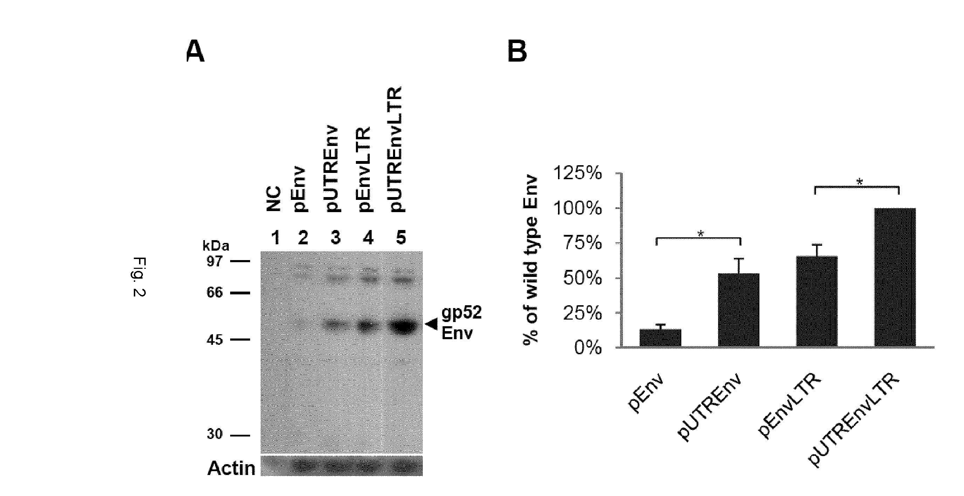

Fig. 2 shows that the 5' leader enhances expression of MMTV Env. (A) An immunoblot analysis using an MMTV Env-specific (anti-gp52/36) polyclonal antiserum was performed to detect Env / gp52 (black arrow) in transiently transfected CrFK cells. Equal amounts (20 µg) of cell extracts were separated by SDS-PAGE, blotted and probed with the MMTV Env-specific antibody (upper panel). Equal protein loading and uniform blotting procedures were followed by membrane stripping and re-probing using an anti-actin polyclonal antibody (bottom panel). NC: mock transfected CrFK cells. (B) Densitometric quantification of the regions corresponding to Env/gp52 was performed using the ImageQuant TL (GE Healthcare). Average quantities +/- standard deviations from two immunoblots are plotted relative to the wild-type construct pUTREnvLTR (set to 100%). A Student's t test was used to assess differences between the UTR-containing and UTR-lacking constructs. * P<0.05.

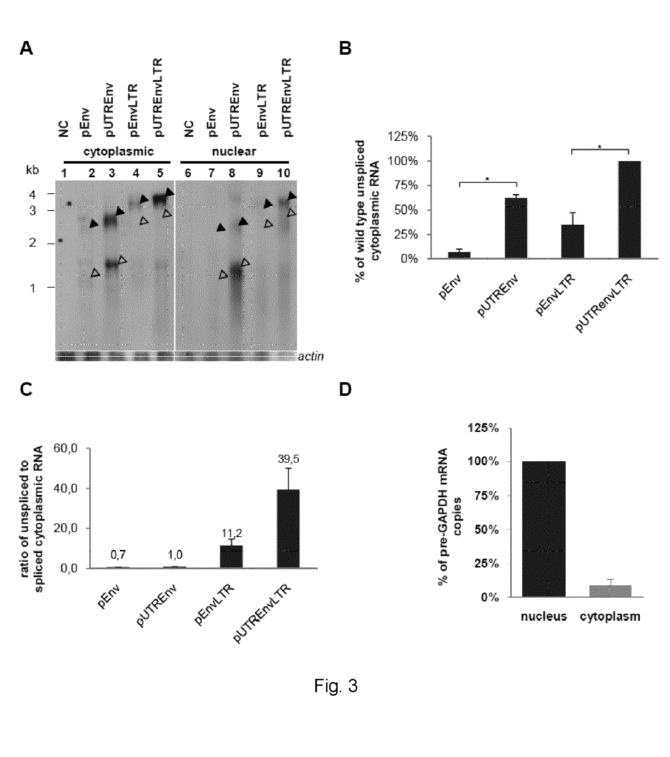

Fig. 3 shows that abundance of the cytoplasmic env mRNA correlates with the presence of the 5' leader. (A) The Env expression constructs, either carrying or lacking the 5' and 3' terminal sequences, were transiently transfected into CrFK cells, the cytoplasmic and nuclear RNA extracted and subjected to agarose gel electrophoresis followed by Northern blot analysis with an env-specific DIG-labelled RNA probe (upper panel). The unspliced env mRNA (black arrow) and spliced rem mRNA (white arrow) transcripts are indicated. After stripping, the membrane was rehybridized with an actin-specific RNA probe to follow RNA loading and a uniform blotting procedure (bottom panel). A representative example from three transient transfection experiments is shown. NC: mock transfected CrFK cells. (B) Signals corresponding to unspliced env mRNA were densitometrically quantified and the obtained quantities were plotted as percentages relative to the wild-type construct, pUTREnvLTR. Average values +/standard deviations from three Northern blot experiments are shown. A Student's t test was used to assess differences between UTR-containing and UTR-lacking constructs. * P<0.001. (C) Unspliced-to-spliced RNA ratio was calculated after quantification of regions corresponding to unspliced env mRNA and spliced rem mRNA. (D) To determine the level of contamination of cytoplasmic fractions with nuclear RNAs, RT-qPCR specific to the intronic regions of feline pre-GAPDH gene (found exclusively in the nucleus) was used. Extracted RNA was treated with TURBO DNase (Ambion) and subjected to RT-qPCR. The cytoplasmic RNA levels are shown as percentage of nuclear RNA levels. Average values +/- standard deviations for all RNA preparations are shown. No amplification was detected when the RT step was omitted.

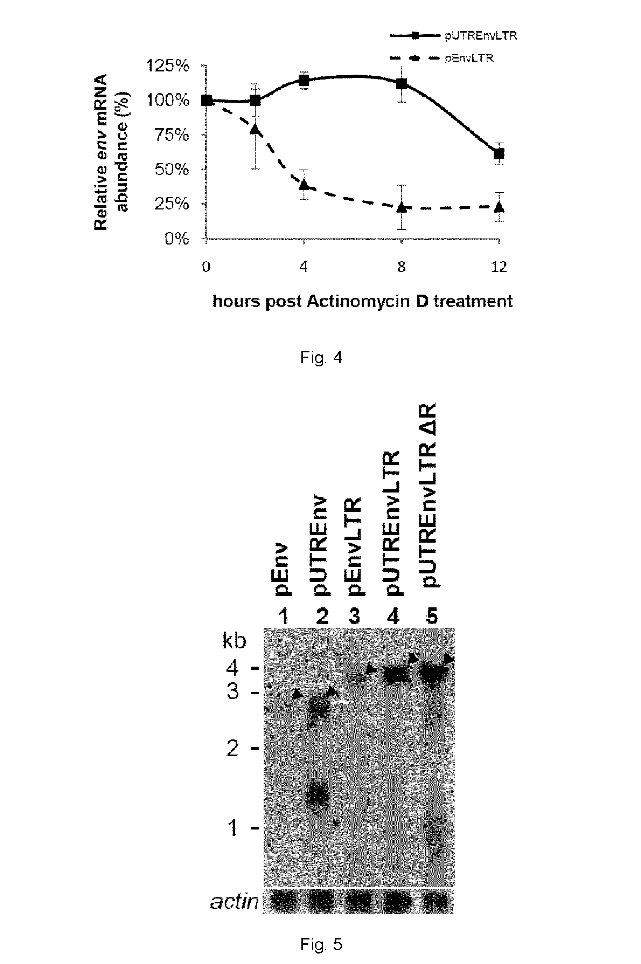

Fig. 4 shows that absence of the 5' leader decreases stability of the env mRNA. Cells transfected with pUTREnvLTR or pEnvLTR, were treated with actinomycin D and the cytoplasmic RNA was extracted at various time points (0 h, 2 h, 4 h, 8 h, 12 h) after the treatment. Northern blotting with an env-specific DIG-labelled RNA probe was used to detect and densitometrically quantify the env mRNA. Black triangles and squares represent relative RNA levels normalized to the actin mRNA levels detected for pEnvLTR and pUTRenvLTR, respectively.
Fig. 5 shows that the R region of the 5' leader is dispensable for augmentation of env mRNA expression. A deletion mutant lacking the R region was transiently transfected into CrFK cells and the env mRNA expression levels were determined by Northern blot analysis using an env-specific mRNA probe (lane 5). Additional Env expression constructs (lanes 1-4) were used for comparison of env mRNA expression levels (upper panel). Black arrows indicate the unspliced env mRNA. The membrane was re-probed with an actin-specific probe (bottom panel).

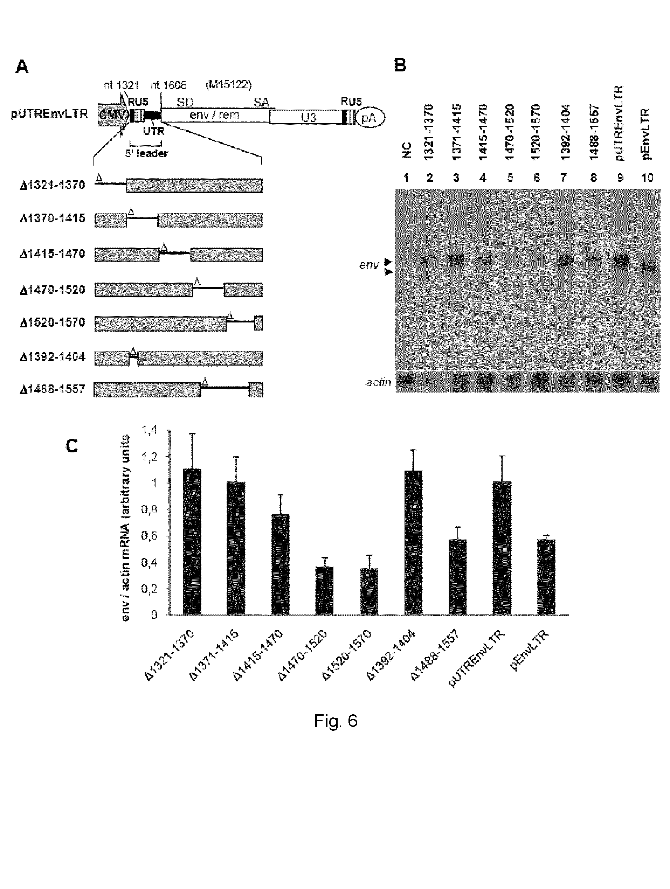

Fig. 6 shows the effect of deletions within the 5' leader on the env mRNA expression. (A) Schematic diagram of pUTRenvLTR and its derivatives lacking an 11-70 bp-long region within the 5' leader sequence (indicated by a horizontal bold line with Δ symbol). Nucleotide positions are according the BR6 strain (GenBank M15122). (B) A representative example from three Northern blot analyses of CrFK cells transiently tranfected with the Env-expression constructs is shown. The cytoplamic RNA extracted from the transfected cells was separated on a denaturing agarose gel, transferred onto a PVDF membrane and hybridized to an env-specific RNA probe labeled with DIG. Re-probing was performed using an actin-specific RNA probe (bottom panel). NC: mock transfected CrFK cells. (C) Densitometric quantification (ImageQuant TL) of env mRNA normalized to actin mRNA levels is shown.

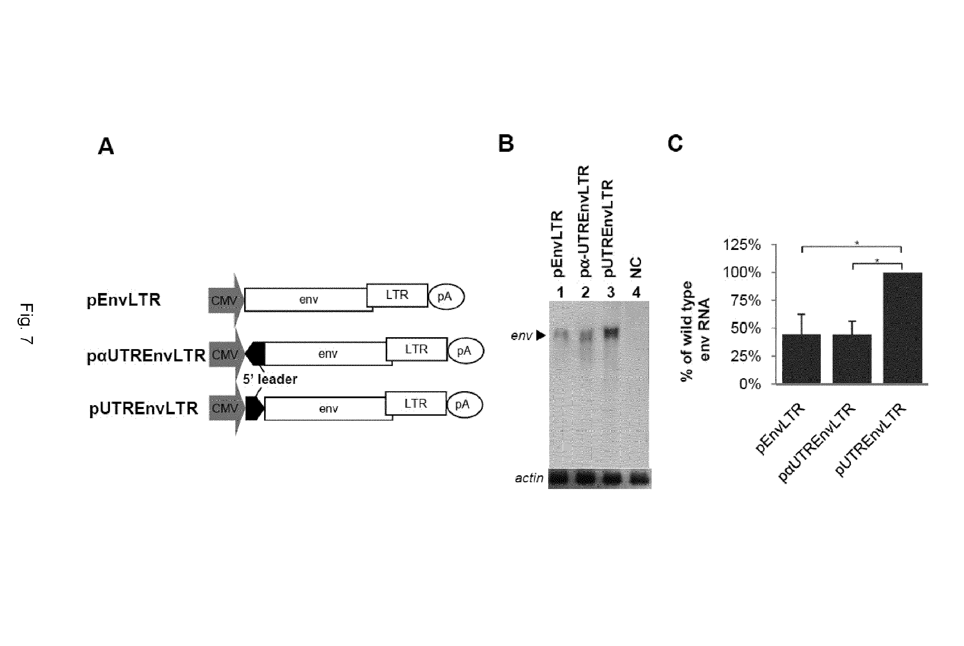

Fig. 7 shows that the 5' leader enhances gene expression when placed in sense orientation. (A) Schematic drawing of the pcDNA3-based constructs used in transient transfection assays. The 5' leader inserted in both orientations into pEnvLTR is depicted as a black pentagon. (B) The Env expression constructs (lanes 1-3) were transfected into CrFK cells. The cytoplasmic RNA was subjected to Northern blot analysis using an env-specific RNA probe together with an anti-DIG antibody and CDP-star chemiluminescent substrate. A representative example of two transfection/Northern blot experiments is shown. An actin-specific RNA probe was used to follow RNA loading (bottom panel). NC: mock transfected cells. (C) The env mRNA levels were densitometrically quantified using ImageQuant TL analysis software and percentages of env mRNA relative to pUTREnvLTR are shown. A Student's t-test was used to evaluate significance of differences between groups. * P<0.05

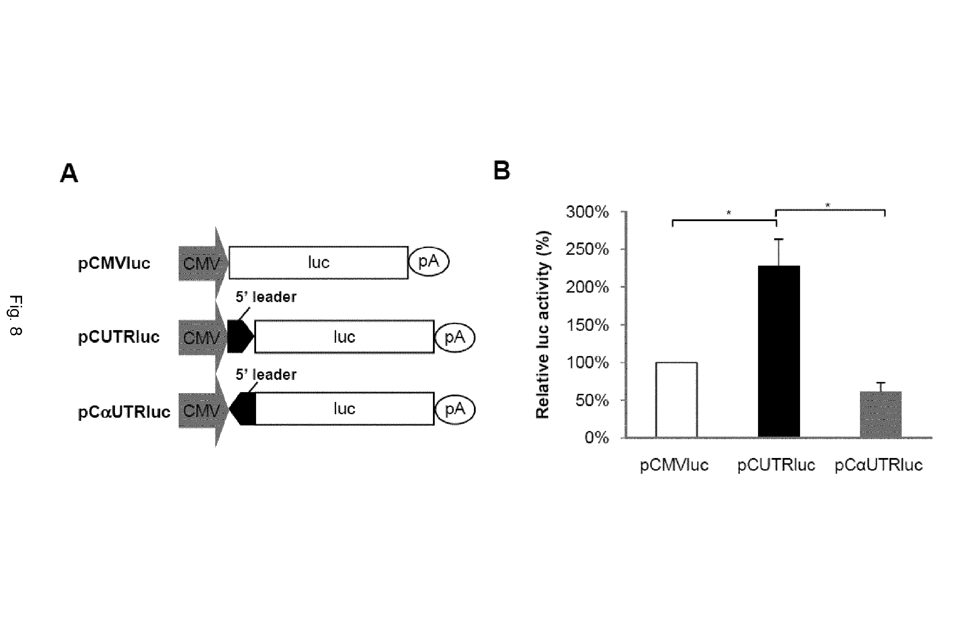

Fig. 8 shows that the luciferase activity is modulated by the 5' leader sequence. (A) Schematic representation of the luciferase expression constructs used for transient transfections. The 5' leader (black pentagon) was cloned in both orientations into the luciferase expression construct, pCMVluc. (B) The luciferase activity in protein extracts was measured 48 h post transfection. Results from three independent transfections are shown as mean luciferase activities relative to transfections with a plasmid lacking the 5'-leader, (pCMVluc; set to 100%; standard deviations are represented by the vertical bars). The statistical significance of differences between groups was tested using an unpaired two-tailed Student's t-test. * P<0.001.

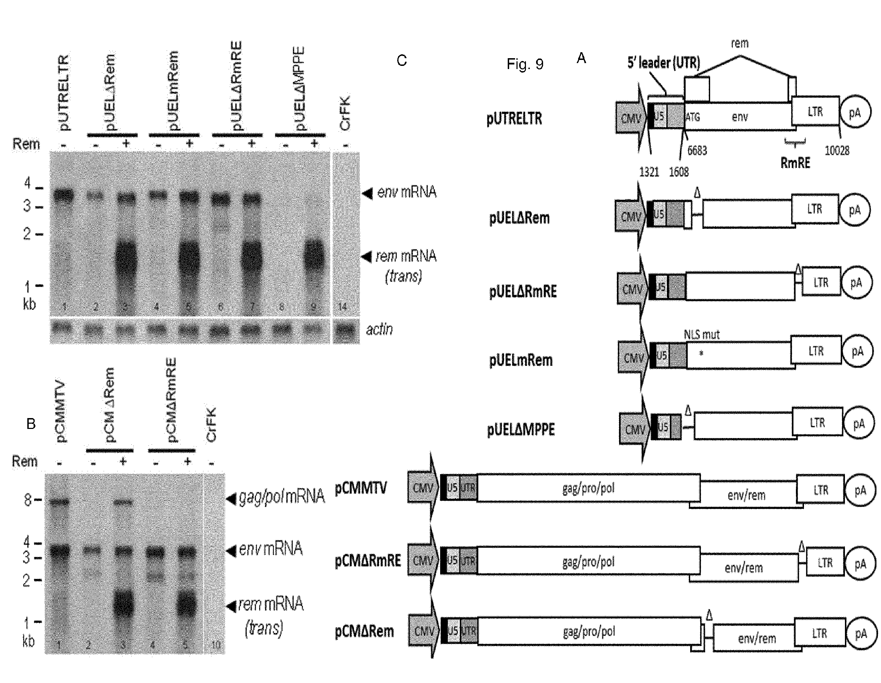

Fig. 9 shows (A) a schematic diagram of the deletion mutants derived either from the subgenomic Env expression construct pUTRenvLTR or from the molecular clone of pCMVMMTV used for transient transfections of CrFK cells. Deletions are marked by a horizontal bold line with Δ symbol. Expression of all constructs is driven by the constitutive CMV promoter. Bovine growth hormone polyadenylation signal (pA) is situated downstream of the 3'LTR. The indicated nucleotide positions are according to the BR6 strain (GenBank Accession no. M15122). Star indicates the mutations introduced to disrupt the nuclear localization signal (NLS) thereby to inactivate activity of Rem (B). (C) The cytoplasmic RNA preparations from transfected cells were analyzed by Northern blot using an env-specific DIG-labelled antisense RNA probe. Where indicated the Rem expression construct was included in transfection. Transfection efficiency was monitored using an EGFP expression plasmid followed by FACS analysis. Equivalent loading and uniformity of blotting was controlled by reprobing the membrane with an actin-specific RNA probe.

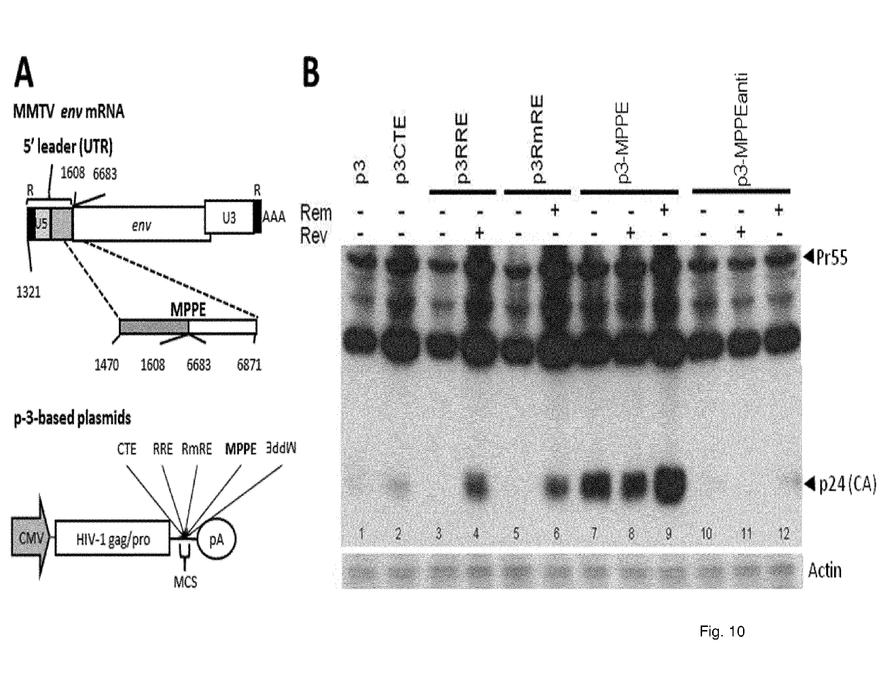

Fig. 10 shows (A) a schematic representation of the MMTV env mRNA and the HIV-1 Gag/PR expression plasmids. (B) Immunoblot analysis of CrFK cells using specific anti-HIV-1 CA antiserum. Cells were transfected with p-3 plasmid or its derivatives carrying either the CTE from MMTV or RRE or RmRE or finally MPPE in either sense or antisense orientation. The Rem and Rev expression constructs, respectively were cotransfected when indicated. The Gag polyprotein Pr55 and p24 (CA) are marked on the right. The additional proteins that migrate between Pr55 and p24 correspond to Gag intermediate cleavage products. Equivalent loading and blotting procedure was controlled by reprobing the membrane with an anti-actin antibody. No significant differences in transfection efficiencies (GFP-positive cells) were detected.

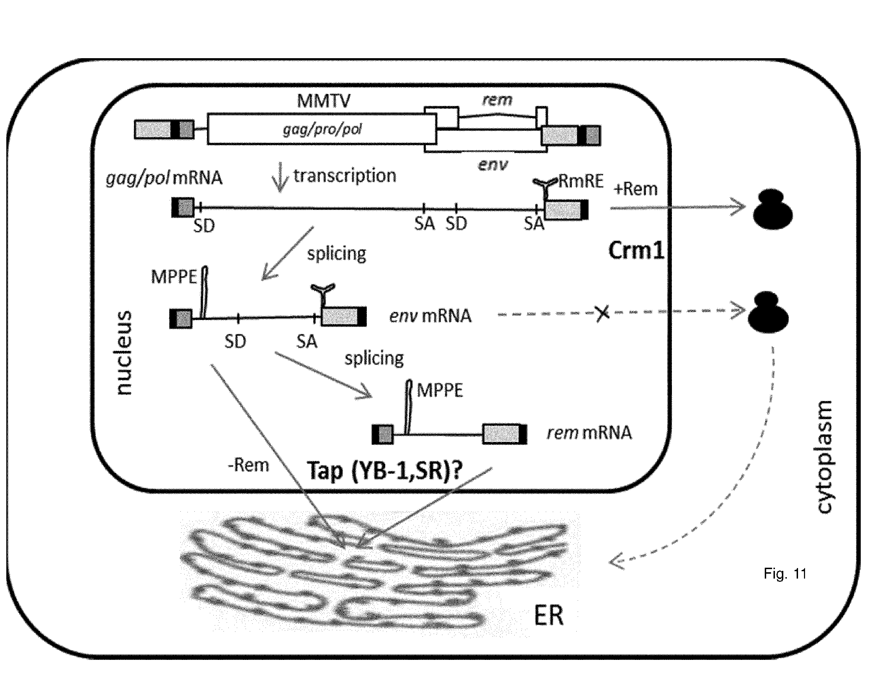

Fig. 11 shows a schematic representation of the transport actions from the nucleus to the cytoplasm.

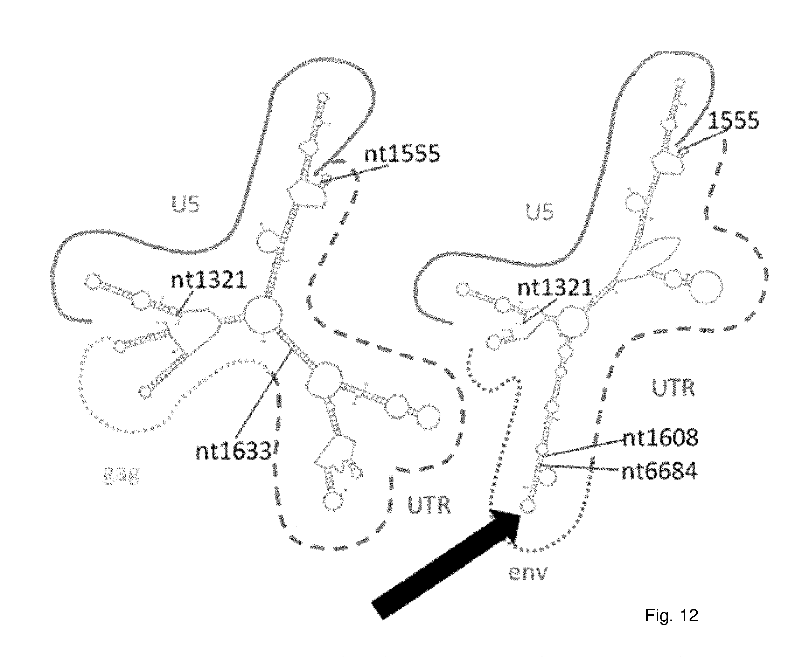

Fig. 12 shows the sequence and predicted structure of the 5' end-proximal regions from the MMTV gag/pol mRNA (left panel) and the MMTV env mRNA (right panel). The 5' terminal sequences encompassing the R, U5 (bold green line), UTR (dashed red line) and either the 5' end of gag (dotted orange line) or the 5' end of env (dotted blue line) were subjected to computer predicition of folding potential using the Mfold program. The most stable predicted structure is shown. Nt coordinates are according to the BR6 strain (GenBank 15122). Arrow indicates a stem-loop predicted to fold in the spliced env mRNA.

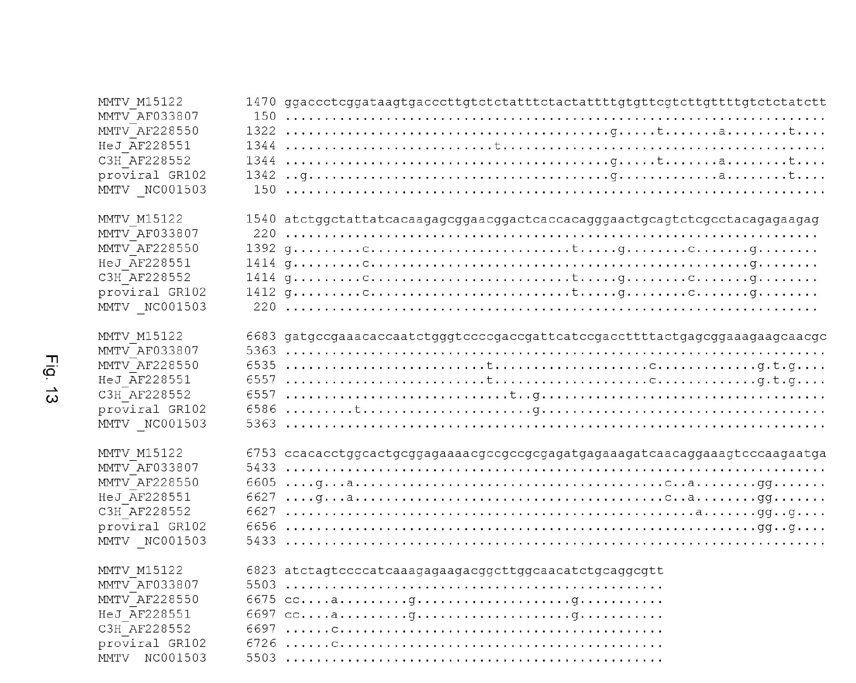

Fig. 13 shows a sequence alignment of MPPE sequences from different MMTV strains (coordinates based on BR6 (M15122) strain.

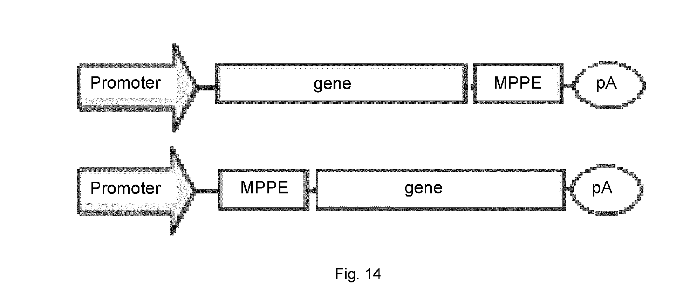

Fig. 14 shows a schematic representation of an expression cassette carrying MPPE according to the present invention in an expression vector or in an isolated nucleic acid molecule (especially DNA).

### EXAMPLES:

### 1.: The 5' Leader Sequence of Mouse Mammary Tumor Virus Enhances Expression of the Envelope and Indicator Genes

### Materials and Methods

### Cell culture, transfections and infections.

The human 2GP19T packaging cell line stably transfected with the MLV Gag/Pol expression construct, pGagPolGpt, have been described elsewhere (Pambalk et al., BBRC 293 (2002), 239-246). The CrFK cell line (CCL-94TM) and 2GP19T cells were cultivated in Dulbecco's modified Eagle's medium with Glutamax (DMEM/GlutaMAXTM-I, Invitrogen Life Technologies/GIBCO, Calif., USA) supplemented with 10% heat inactivated bovine fetal serum (PAA Laboratories, Austria). Transfections of CrFK cells were performed as described in (Mullner et al., NAR 36 (2008), 6284-6294). Transfection efficiency with constructs carrying the Env expression cassette was determined by measuring the luc gene expression after co-transfection of pCMVluc (0.5 µg). For the luciferase expression analysis, 700 ng of pCMVluc, pCUTRluc and pCαUTRluc was used. 2GP19T cells were co-transfected with an MLV-based vector, pLXSN-EGFP (2.5 µg, (Klein et al., Gene Ther. 4 (1997), 1256-1260) and equimolar amounts of the Env expression plasmids (adjusted to 1.8 µg with pcDNA3) by calcium phosphate coprecipitation as recommended by the supplier (CellPhect Transfection Kit, GE Healthcare UK Limited, UK). Forty-eight hours after transfection, the virus-containing cell culture supernatant was clarified by a low-speed centrifugation and filtered through a 0.45 µm filter (Filtropur S, SARSTEDT, Numbrecht, Germany). The filtered supernatant containing polybrene (8 µg/ml, Sigma-Aldrich, Inc., Austria) was used to transduce 4 x 10⁵ CrFK cells seeded in 6-well culture plates. The transfected and infected cells were analyzed by FACS to measure the transfection and infection efficiencies, respectively.

### Plasmid construction.

The construction of the Env protein expression plasmids pEnvLTR and pEnv has been outlined previously (Indik et al., Virology 337 (2005a) 1-6; Mullner et al., 2008). To introduce the 5' leader (nt 1321 - nt 1608 (position coordinates according to GenBank M15122)) into pEnvLTR the following strategy was used. Total RNA was extracted from CrFK cells infected with a recombinant MMTV-EGFP virus (Indik et al., Cancer Res. 65 (2005b), 6651-6659) using RNeasy Mini Spin Columns (Qiagen GmbH, Austria). After reverse transcription performed using SuperScript II reverse transciptase (Invitrogen Life Technologies, Austria) with a MMTV-specific reverse primer 9877RXho (5'-AAA AAA CTC GAG TCA GCA CTC TTT TAT ATT ATG G-3'), the obtained cDNA was amplified by PCR (High Fidelity PCR System, Roche Diagnostics GmBH, Mannheim, Germany). The following primer pair was used for amplification: 1321FHind (5' -AAA AAA AAG CTT GCA ACA GTC CTA ACA TTC AC-3') and MMTVenvR1 (5'-AAA AAA TCT AGA CTA AGT GTA GGA CAC TCT CGG -3'). A smaller amplification product of ∼2.3 kb, representing the single spliced env mRNA, was extracted from an agarose gel and digested with HindIII and BamHI restriction enzymes, introduced by the oligonucleotides (underlined; Promega GmbH, Austria). The HindIII - BamHI fragment was then introduced into the corresponding sites in pEnvLTR. An analogous approach was used for the generation of pUTREnv. In this case, the PCR product was digested at the HindIII and XbaI restriction sites and cloned into the multiple cloning site of the pcDNA3 (Invitrogen, Life Technologies, Austria). A vector containing the 5' leader lacking the R region, pUTREnvLTRdelR, was prepared by inserting a truncated 5' leader sequence into pEnvLTR. The truncated 5' leader was generated by PCR amplification using primer 25FHind (5' -AAA AAA AAG CTT ATT CAC CTC TCG TGT GTT TGT GTC -3'; HindIII site underlined) in combination with MMTVenvR1 and the plasmid pUTREnv as a template. The HindIII - Bam HI fragment was cloned into pEnvLTR using the relevant restriction enzymes. Deletion of 10-70 bp long fragments of the 5' leader from pUTREnvLTR was obtained by a Long template PCR-based approach (Expand Long Range, dNTPack, Roche Diagnostics GmbH, Mannheim, Germany) using primers described in Supplementary data. Amplification products were digested with EcoRI and circularized. Construction of pUTRluc and pαUTRluc was performed as follows. The 5' leader flanked by the HindIII restriction sites was generated by high fidelity PCR amplification using a forward primer 1321FHind (5'-AAA AAA AAG CTT GCA ACA GTC CTA ACA TTC AC-3') in combination with a reverse primer UTRRHind (5' -AAA AAA AAG CTT CTC TTC TCT GTA GGC GAG AC-3') and pUTRenv as a template. The HindIII-digested fragment (HindIII restriction site is underlined) was then inserted into the corresponding restriction site upstream of the luciferase gene in pCMVluc. Orientation of the insert was verified by restriction analysis followed by sequencing.

### Immunoblot analysis.

Immunoblot analysis was performed as described previously (Indik et al., 2005a).

### RNA extraction and Northern blot analysis.

Preparation of cytoplasmic and nuclear RNA fractions as well as Northern blot analysis was described previously (Mullner et al., 2008). Sub-cellular fractionation was controlled using real time RT-PCR (Power SYBR Green RNA-to-CT 1-Step kit; Applied Biosystems GmbH, Austria) with primers homologous to intron 6 (f-pre-GAPDH fw: 5'-TAC GGG TGA TGG GGA AAG-3'; f-pre-GAPDH rv: 5'-TCC TCC CCT GGG AAG ACT TG-3') of the unprocessed pre-mRNA of the glyceraldehyde-3-phosphatase dehydrogenase (pre-GAPDH mRNA) (Blissenbach et al., J. Virol. 84 (2010), 6598-6604). Extracted cytoplasmic and nuclear RNAs were treated with TURBO DNase (Ambion/Applied Biosystems GmbH, Austria). Two hundred nanograms of RNA and serial dilutions of the nuclear RNA were subjected to real-time RT-PCR. Subsequently, percentage of pre-GAPDH mRNA in the cytoplasm relative to nuclear pre-GAPDH mRNA level was calculated. Stability of mRNA transcripts was assessed after actinomycin D treatment of CrFK cells transiently transfected with pUTREnvLTR and pEnvLTR, respectively. Actinomycin D (10 µg/ml; Sigma-Aldrich, Inc.) was added to the transfected cells 48 h post transfection. The cytoplasmic RNA was extracted from the transfected cells 0 h, 2 h, 4 h, 8 h and 12 h after the treatment and subjected to Northern blot analysis. The unspliced env mRNA and spliced rem mRNA was quantified using ImageQuant TL analysis software (GE Healthcare UK Limited, UK) and normalized to actin mRNA levels.

### Luciferase reporter assay.

CrFK cells grown in 6 well plates were analyzed 48 h after transfection using the Bright-Glo Luciferase assay system (Promega GmbH). Parallel cultures were assayed for transfection efficiencies by FACS analysis (pCMV-EGFP was co-transfected with luciferase expression constructs). Cells were rinsed once with phosphate-buffered saline (PBS) and disrupted in 500 µl Glo Lysis buffer (5 min, room temperature, rocking platform). 100 µl of Bright Glo assay reagent was added to the same volume of the lysate and luminescence measured using a Tecan GENIOS reader (Tecan Group Ltd., Switzerland). An unpaired two-tailed Student's t-test was used to determine whether the differences in luciferase activities were statistically significant.

Results:
The presence of the 5' leader region augments production of MMTV-Env pseudotyped MLV virions.

To test whether the 5' terminal sequences are able to modulate gene expression, a series of MMTV-Env subgenomic expression constructs was generated (Fig. 1A). All the plasmids contain the MMTV env gene under the transcriptional control of the heterologous cytomegalovirus (CMV IE) promoter, allowing constitutive, dexamethasone-independent, expression. The expression constructs contain the standard splice donor and acceptor sites for rem mRNA (the spliced rem mRNA is exported from the nucleus via the standard pathways for spliced mRNA (Indik et al., 2005a; Mertz et al., 2005)). MMTV Env expression was first tested using the ability of the MMTV Env to pseudotype MLV particles (Golovkina et al., J. Virol. 72 (1998), 3066-3071). The Env-expression plasmids, together with the MLV-based vector pLXSN-EGFP, were transfected into a semi-packaging cell line, 2GP19T, expressing MLV gag and pol genes (Pambalk et al., 2002). The expression of MMTV Env results in the production of EGFP-containing MMTV pseudotypes capable of infecting MMTV-permissive feline kidney cells (CrFK) (Gunzburg et al., Virology 155 (1986), 236-248; Howard et al., Cancer Res. 37 (1977), 2696-2704; Indik et al., 2005b). Transfection of all four Env expression constructs resulted in infection of CrFK cells (Fig. 1B). Although transfection efficiencies, measured by FACS analysis of transfected cells, were not significantly different, marked differences in the number of transduced cells were detected (Fig. 1B). The lowest level of infection (4.2 × 10² +/- 0.9 × 10¹ GFU/ml (average for two infection experiments +/- standard deviation)) was obtained with the construct bearing the env open reading frame (ORF) alone (Fig. 1B, pEnv). An approximately two-fold increase in infection levels was observed with the Env expression construct containing the 5' leader region (pUTREnv). The presence of the 3' LTR had a similar positive effect on transduction efficiency (pEnvLTR). The highest increase in virus titers was obtained with the construct bearing both regions flanking the env ORF (Fig. 1B, pUTREnvLTR; 3 × 10³ +/- 0.8 × 10² GFU/ml). Thus the results from the pseudotyping/transduction experiments show that the 5' leader and the 3'LTR have an effect on Env expression.

Differences in pseudotype transduction efficiencies are attributable to differences in Env protein levels.

To investigate whether the differences in infection levels are due to differences in the abundance of the Env protein, CrFK cells transfected with the Env expression constructs were subjected to an immunoblot analysis. The signal intensities of the obtained bands were densitometrically quantified and normalized to the level of actin protein (Fig. 2A & B). The minimum level of the Env protein is produced in cells transfected with pEnv. When either the 5' leader or the 3' LTR were included into the expression plasmids, a substantial increase in amounts of Env was reproducibly observed, indicating that each of these elements has a marked stimulatory effect on protein expression (Fig. 2A, lanes 3 and 4; pUTRenv, pEnvLTR). A further modest (∼two-fold) increase in Env levels was detected, when both env gene-flanking regions were included in the expression construct (Fig. 2A, lane 5; Fig. 2B; pUTREnvLTR). Collectively, results from the transduction and immunoblot blot analyses indicate that the 5' leader and the 3'LTR sequences significantly modulate MMTV Env protein production.

### The presence of the 5' leader increases steady-state env RNA levels.

To investigate whether the changes in MMTV Env production could be attributed to changes in steady-state mRNA levels or to an increased translation rate, a Northern blot analysis of cells transfected with the Env expression constructs was performed. Cytoplasmic and nuclear RNAs were isolated 48 h after transfection and analyzed using an antisense DIG-labelled env probe corresponding to the N-terminal portion of Env. For MMTV, the standard wild-type env mRNA (corresponding to the mRNA expressed from the pUTREnvLTR plasmid) and spliced rem mRNA are 3.8 kb and 2.6 kb, respectively. Deletion of the 5' leader and the 3'LTR results in production of mRNA which is 0.3 kb and 1.3 kb smaller in size, respectively, as expected. Cytoplasmic and nuclear fractionation was monitored using a pre-GAPDH-specific real time RT-PCR (Blissenbach et al., 2010). The same amounts of extracted nuclear and cytoplasmic fractions were subjected to real-time RT-PCR with primers spanning the intronic sequences of the feline pre-GAPDH. Quantification of the real-time RT-PCR products showed that on average less than 9% (8.6% +/- 4.6%) of the extracted cytoplasmic RNA was derived from the nucleus (Fig. 3D). Results from triplicate transfection/Northern blot analyses revealed marked differences in the amounts of unspliced env mRNA. Readily detectable env mRNA was present in the cytoplasmic fraction derived from cells transfected with the wild-type Env expression construct (pUTREnvLTR, Fig. 3A and B, set to 100%). In sharp contrast, transfection with pEnv (plasmid lacking the 5' leader and the 3'LTR) resulted in less than 7% of env mRNA levels being detected compared to the wild-type transcript (Fig. 3A and B). Significantly reduced cytoplasmic env mRNA levels were also detected with constructs lacking either the 5' leader (pEnvLTR) or the 3'LTR (pUTRenv). Quantification of the unspliced env mRNA showed an approximately 65% (pEnvLTR) and 40% (pUTRenv) reduction to that of the wild-type pUTREnvLTR construct (Fig. 3A and B).

Of particular interest were elevated env mRNA levels resulting from transfections with plasmids containing the 5' leader. Whereas transfection with the plasmid containing only the Envcoding sequences, pEnv, resulted in the minimum env mRNA levels (6.7% +/- 3.3% (average +/- standard deviations) of that of the wild type), the presence of the 5' leader (pUTREnv) significantly elevated cytoplasmic env mRNA levels (Fig. 3A, compare lanes 2 and 3; Fig 3B, 61.9% +/- 3.5% compared to wild type). Similarly, cytoplasmic fractions of cells transfected with the 5' leader-containing construct pUTREnvLTR showed a three- to four-fold increase in env mRNA levels of that of the 5' leader-lacking construct pEnvLTR (Fig. 3A, compare lanes 4 and 5; Fig. 3B). These results indicate that the 5' leader has a positive effect on cytoplasmic env mRNA accumulation. Moreover, the presence of relatively high levels of the unspliced mRNA lacking the 3'LTR (derived from and pUTREnv plasmid) in the cytoplasm suggests that this mRNA contains a cis-acting element allowing nucleo-cytoplasmic translocation of mRNA.

Interestingly, Northern blot analyses also revealed significant differences in the amounts of the spliced rem mRNA. Cytoplasmic extracts from cells transfected with plasmids containing the 3'LTR (pEnvLTR and pUTRenvLTR) contained extremely low levels of the spliced mRNA (Fig. 3A). The calculated ratio of unspliced to spliced mRNA was 11.2 for pEnvLTR and 39.5 for pUTREnvLTR (Fig. 3C). Conversely, a significant portion of mRNA present in the cytoplasm of the pUTREnv- and pEnv-transfected cells represented the spliced rem mRNA (Fig. 3A, lanes 2 and 3), with the ratio of unspliced to spliced mRNA was substantially reduced, reaching values of 0.7 and 1.0 for pEnv and pUTREnv, respectively (Fig. 3C). Thus, the presence of the 3'LTR but not the 5' leader appears to efficiently interfere with the cellular splicing apparatus.

These Northern blot results show that the 5' and 3' terminal sequences have a significant positive effect on the expression of the MMTV env mRNA. The presence of the 3'LTR interferes with the activity of the cellular splicing apparatus and allows, to some extent, nucleo-cytoplasmic translocation of the unspliced env mRNA. The 5' leader sequence of MMTV has a profound effect on the steady-state abundance of cytoplasmic mRNA, showing that this region contains a cis-acting element affecting extra-nuclear localization of mRNA, although increased stability of env mRNA could also be playing a role. Taken together, the present data show that the high level of Env protein expression mediated by the pUTREnvLTR construct is not exclusively attributable to an increased translational utilization of env mRNAs.

### The 5' leader enhances mRNA stability.

To better define mechanism by which the 5' leader enhances steady-state mRNA levels, the stability of env mRNA carrying or lacking the 5' leader sequence was tested. To this end, CrFK cells were transfected with pUTREnvLTR or pEnvLTR constructs. The cells were treated with actinomycin D, an inhibitor of transcription, 48 h post transfection and subsequently cytoplasmic RNA was isolated for up to 12 h after actinomycin D treatment. Northern blot analysis combined with quantification of the env mRNA showed pronounced differences in stability of the two respective mRNA molecules that differ only in their 5'terminal sequences. While the env mRNA containing the 5' leader (pUTREnvLTR) was degraded only to a minor extent 8 h after addition of actinomycin D, the absence of the 5' leader (pEnvLTR) resulted in a more than 50% decrease in the env mRNA as early as 4 h after actinomycin D treatment (Fig. 4). Therefore, the 5' terminal sequence stabilizes the env mRNA and the observed up-regulation of env gene expression results, at least in part, from this stabilisation effect.

### The 3' end of the 5' leader enhances gene expression.

To localize the MMTV sequences responsible for the observed enhancement of env gene expression, a deletion scanning mutagenesis analysis was performed. A series of deletion mutants each lacking an 11-70 bp-long portion of the 5' leader sequence was constructed (Fig. 6A), the respective plasmids transfected into CrFK cells and cytoplasmic mRNA levels determined by Northern blot analysis. A representative example of three transfection/Northern blot experiments is shown in Fig. 6B. The mRNA levels (normalized to actin levels) produced by the deletion mutants ΔUTR1321-1370, ΔUTR1371-1415, ΔUTR1415-1470, ΔUTR1392-1404 were similar in magnitude to the RNA levels produced from the wild-type construct pUTREnvLTR (Fig. 6B, lanes 2-4 and 7; Fig. 6C). In contrast, a marked reduction in mRNA yield was observed for three mutants (ΔUTR1520-1570, ΔUTR1488-1557, ΔUTR1470-1520) lacking the distal part of the 5' leader in a close proximity to the Env translation start codon (Fig. 6B, lanes 5, 6 and 8, Fig. 6C). In this case, the env mRNA levels were equal to or smaller than the levels determined for the UTR-lacking plasmid, pEnvLTR. These results show that the 3'proximal end rather than the 5' proximal end and central region of the 5' leader have a positive effect on the cytoplasmic env mRNA steady-state levels.

The 5' leader sequence-mediated effect on gene expression is orientation dependent and the 5' leader augments expression of the non-viral luciferase gene.

To address the question of whether the 5' leader sequence functions in an orientation-dependent manner, the 5' leader region was inserted in both orientations into the Env expression construct, pEnvLTR (Fig. 7A; sense: pUTREnvLTR; antisense: pαUTRenvLTR). Results from two Northern blot analyses show an approximately three-fold decrease in env mRNA levels (compared to wild-type pUTREnvLTR) in cytoplasmic extracts from cells transfected with the construct carrying the 5' leader in reverse orientation (Fig. 7B and 7C). The env mRNA levels determined for this plasmid were similar to the env mRNA levels detected for the construct lacking the 5' leader, pEnvLTR (Fig. 7A and B). Therefore, the 5' leader functions in an orientation-dependent manner to enhance cytoplasmic accumulation of env mRNA.

To further confirm these results, as well as to determine whether the activity of the 5' leader is dependent on the presence of an additional MMTV-encoded factor, the 5' leader was inserted in both orientations upstream of a heterologous firefly luciferase gene. The resulting plasmids pCUTRluc (5' leader in sense orientation) and pCαUTRluc (antisense orientation) were transfected into CrFK cells (Fig. 8) and luciferase activity determined 48 h post transfection. Results from triplicate transfection experiments showed that the 5' leader in the inverted orientation did not increase luciferase expression compared to a construct lacking the 5' leader, pCMVluc (Fig. 8B). In contrast, a two- to three-fold increase in luciferase activity was consistently detected with the plasmid pCUTRluc containing the 5' leader in sense orientation (Fig. 8B). The magnitude of the increase seems to be proportional to the increase observed in Northern blot analyses. Therefore, the 5' leader per se is sufficient to confer a positive effect on gene expression when placed in sense orientation.

### Discussion:

The goal of this study was to define whether the MMTV RNA region, previously predicted to be extensively folded and localized within the 5' leader sequence, influences MMTV env gene expression. To this end, the 5' leader sequence was inserted into an MMTV Env expression construct and a luciferase reporter vector, and protein and mRNA levels investigated and compared to controls lacking this region. By these means it was demonstrated that the 5' leader has indeed a positive effect on MMTV as well as luciferase gene expression. Both Env production and luciferase activity were increased in the presence of this element placed under the control of the strong CMV IE promoter/enhancer. The magnitude of this increase was proportional to that observed previously for a posttranscriptional control element (PCE) located in the RU5 region of spleen necrosis virus (SNV) stimulated luciferase expression by about 1.7-fold when placed downstream of the CMV IE promoter. The PCE is a stem-loop RNA structure initially identified within the 5' leader sequence of SNV as a sequence which has a positive effect on gene expression. A growing collection of retroviruses, including Mason-Pfizer monkey virus (MPMV), human foamy virus (HFV), reticuloendotheliosis virus strain A (REV-A), human T-cell leukemia virus type 1 (HTLV-1), feline leukemia virus (FeLV) and bovine leukemia virus (BLV) was subsequently reported to contain a PCE. Quantitative RNA analysis together with ribosomal sedimentation profile assays revealed that this sequence acts at the posttranscriptional level by inducing robust translation of PCE-containing mRNA. The translational enhancement is due to the interaction of the PCE with RNA helicase A, allowing the translation-inhibitory effect of the highly structured 5' leader to be overcome and facilitating polysomal association with the mRNA, ultimately leading to an increased protein production. Analogous to the PCE, the 5' leader of MMTV augments gene expression only when inserted in sense orientation (Fig. 7B and 7C; 8B). However, in contrast to the PCE-mediated effect, the present analysis demonstrates a profound effect of the 5' leader on the cytoplasmic mRNA levels. Therefore, the possibility was excluded that the detected increase in gene expression is solely attributable to a translational enhancement. Instead, the present experiments show that the 5' leader functions as a multimodal region, which affects gene expression mainly at the pre-translational level. In particular, it was shown that this region supports extra-nuclear localization and enhances the stability of the env mRNA transcripts. The effect of the 5' leader on MMTV mRNA nucleo-cytoplasmic transport is of particular interest because the native env mRNA has the capacity to encode Rem and contains the RmRE. Therefore, one would anticipate that the Crm1-dependent pathway would be involved in regulation of the cytoplasmic env mRNA levels. It appears, however, that Rem and RmRE are dispensable for the extra-nuclear accumulation of the env mRNA. Previously, it was shown that unlike the unspliced full length viral RNA, which is exported from the nucleus only in the presence of Rem/RmRE, deletion of either Rem or RmRE (or both) has only a moderate effect on the export of the env mRNA from the nucleus. Furthermore, treatment of cells with leptomycin B, a drug, which specifically blocks the CRM1-dependent RNA export pathway, markedly reduced cytoplasmic levels of the unspliced viral RNA but the env mRNA levels remained virtually unchanged. These data show that the env mRNA, which may be internally spliced (giving rise to the rem mRNA), is able to subvert the normal cellular pre-mRNA processing/splicing machinery and achieves nuclear export via a Rem-independent mechanism. The present results show that the 5' leader is involved in cytoplasmic accumulation of the env mRNA.

A positive posttranscriptional effect of the 5' leader has previously been also documented for a closely related betaretrovirus, JSRV, encoding a Rev-like protein, Rej. Analogously to MMTV, a synergistic positive effect of the JSRV 5' leader and the 3' LTR on the env gene expression has been reported. Increased cytoplasmic steady-state env mRNA levels resulted from an increased nuclear export, enhanced RNA stability and controlled splicing. The combination of these effects ultimately resulted in greater envelope protein levels and higher virus/vector titers. Furthermore, analogously to MMTV, Rej activity has been found to be dispensable for cytoplasmic accumulation of the env mRNA and blocking of the Crm1-dependent RNA export pathway did not inhibit Env expression. Therefore, one may envisage that both betaretroviruses employ similar strategies to regulate expression of the unspliced and the single spliced mRNAs, which differ from that employed by HIV-1. Whereas in the case of HIV-1 Rev mediates nucleo-cytoplasmic translocation of the unspliced as well as the single spliced mRNAs, Rem (Rej) seems to primarily affect export the unspliced viral RNA.

The present experiments further show that, in addition to the mRNA trafficking, the 5' leader influences mRNA stability. The half-life of RNAs is an intrinsic property determined by their rate of decay. The stability of any mRNA may be indirectly influenced by mRNA localization. A direct mechanism of RNA degradation commonly involves a sequence- or structure-specific endoribonuclease-recognizing cis-acting cleavage sites. Deletion mutagenesis, used to define sequence boundaries necessary for the 5' leader activity, identified the distal part of the 5' leader as the region responsible for the increased gene expression. Thus, it is evident that the distal part of the 5' leader is involved in the interaction with RNA-binding proteins stabilizing mRNA analogously to the previously demonstrated regulation of the transferrin receptor expression. Furthermore, it is also conceivable that the distal part of the 5' leader adopts a conformation that is more resistant to ribonucleases or drives translocation of mRNA to a cellular compartment lacking (or containing less active) endonucleases.

These results provide evidence that the 5' leader of MMTV increases the steady-state mRNA abundance. Although additional mechanisms cannot be ruled out, it is evident that the observed enhancement of gene expression results primarily from a combined effect of the 5' leader on mRNA stability and nuclear export. The identification of the region responsible for the augmentation of mRNA levels further underlines the importance of the 5' leader in the life cycle of the prototypic betaretrovirus associated with mammary adenocarcinomas in mice and possibly involved in human breast carcinogenesis and some hepatic disorders. This element further broadens the list of functional domains that have been identified within the 5' leader sequence of betaretroviruses and further emphasizes different strategies that these viruses employ to control gene expression.

2.: Mutations or deletions within either the MMTV rem coding region or the RmRE do not abrogate cytoplasmic accumulation of env mRNA
During attempts to produce the MMTV envelope protein (Env) from subgenomic expression constructs either containing or lacking the 5' leader sequence, it was observed that the 5' leader markedly enhances Env expression. The presence of the 5' leader increased stability and enhanced export of the env mRNA from the nucleus. The region responsible for this effect has been mapped to the distal part of the 5' leader sequence. This result was rather surprising since the Env expression construct has the capacity to encode Rem (env and rem ORFs overlap) and carries the RmRE (Fig. 9A). To further confirm these data and to demonstrate that the env mRNA is exported from the nucleus in Rem-independent manner, a series of Env expression plasmids was constructed carrying either mutations in the nuclear localization sequence of Rem (NLS; this mutation abrogates the nuclear localization of Rem) or deletions within either the Rem coding region or the RmRE. Furthermore, deletions in the Rem-coding region or RmRE were also introduced into the molecular clone of MMTV (pCMMTV) (Fig. 9A). Consistent with the previous results, Northern blot analysis of the cytoplasmic RNA fractions revealed that deletion of Rem from the molecular clone dramatically decreased cytoplasmic unspliced gag/pol mRNA levels (Fig. 9B; lane 2). In sharp contrast, only a small decrease in the env mRNA levels was detected (Fig. 9B; lane 2). The expression of the cytoplasmic gag/pol mRNA from pCMΔRem was rescued when Rem was expressed in trans (Fig. 9B; lane 3). Similarly, the deletion of RmRE from the molecular clone (pCMΔRmRE) resulted in a loss of the unspliced mRNA, whereas the env mRNA levels remained virtually unchanged (Fig. 9B; lanes 4). In this case, the expression of the gag/pol mRNA could not be rescued by expression of Rem (Fig. 9B; lane 5).

Experiments performed using subgenomic Env expression constructs (pUTRELTR and its derivatives; Fig.9A) revealed that the deletion of RmRE has no effect on cytoplasmic env mRNA levels (Fig. 9C; lane 6). Analogously to the experiments with the molecular clone only a moderate decrease in env mRNA was detected when either a 100 bp-long region in the rem coding sequence was deleted (pUELΔREM, Fig. 9C; lane 2) or when the NLS of Rem was mutated (pUELmREM, Fig. 9C; lane 4). Importantly, deletion of a region spanning the 5'UTR-env junction resulted in a complete loss of the cytoplasmic env mRNA regardless of whether the Rem expression plasmid was cotransfected or not (Fig. 9C, lane 8 and 9). These results showed that the region spanning the 5'UTR-env junction (MPPE) significantly affects cytoplasmic env mRNA abundance. Furthermore, these results also showed that the env mRNA can be exported from the nucleus in an Rem/RmRE independent manner.

### 3.: MMTV fragment coinciding with the UTR-env junction functions as a cis-acting element substituting for Rev /RRE in a HIV-Gag/PR subgenomic expression construct

To identify a positive-acting factor responsible for the cytoplasmic accumulation of env mRNA, a reporter gene construct derived from the HIV-1 Gag/PR expression plasmid 3-(-) (Wodrich et al., J. Virol. 75 (2000), 10670-10682) was employed. The HIV-1 gag contains regions collectively referred to as cis-acting inhibitory sequences (INS), which account for nuclear retention and instability of intron-containing HIV-1 mRNAs. The interaction of Rev with RRE counteracts the effect of INS. Importantly, the Rev/RRE-driven regulation can be replaced by alternative posttranscriptional control elements and this property has been used to identify a number of cellular and viral posttranscriptional control elements including the constitutive transport elements (CTE) from MPMV and SRV-1, RNA transport element (RTE) from intracisternal A-particles (IAP) and a 100 nt sequence regulating nuclear export of intronless histone H2A mRNA.

In view of the previous observation showing that the distal part of the MMTV 5'UTR sequence enhances the steady-state cytoplasmic env mRNA levels (Hohenadl et al., 2011), a 328-bp long fragment corresponding to the 3' part of the 5'UTR and the 5'- end proximal region of the env gene (UTRenv) was introduced in sense or in antisense orientation into the 3-(-) plasmid (nt 1470 - nt 1608 from 5'UTR and nt 6683 - nt 6871 from env) (Fig. 10A). The resulting construct, named p3-MPPE, was transfected into feline kidney cells (CrFK) and the expression of HIV-1 Gag was detected by immunoblot analysis using an anti-p24 antiserum. As controls, plasmids carrying the CTE of MPMV, the RRE of HIV-1 and the RmRE of MMTV were used. Transfection efficiency was monitored using a GFP-expression plasmid, pCMVeGFP. As expected results from the immunoblot analysis of cells transfected with the construct carrying the CTE revealed a signal corresponding to the Pr55gag polyprotein, the proteolytically cleaved CA (p24) and two intermediate cleavage products (Fig. 10B; lane 2). A comparable but slightly stronger signal was detected in cells transfected either with the RRE- or RmRE-containing construct. However, in these cases the p24 expression was only observed when the corresponding auxiliary protein was cotransfected into the target cells (Fig. 10B; lanes 3-6). Importantly, cells transfected with p3-MPPE exhibited a strong HIV-1-specific expression, irrespective of the presence of Rev or Rem (Fig. 10B; lanes 7-9). In contrast, no p24 was observed when the same sequence was present in antisense orientation (Fig. 10B; lanes 10-12), showing that MPPE contains an orientation-dependent cis-acting RNA element that can functionally substitute for the HIV-1 Rev/RRE system.

To determine the species tropism of MPPE, the activity of the HIV-1 Gag/PR-based indicator plasmids upon transfection into the feline cell line CrFK, the murine NIH3T3 cell line or the human cells lines 293T and HeLa were compared. Results from immunoblot analysis show that MPPE is active in all four cell lines. Increased p24 levels were determined in cells transfected with the MPPE-containing construct compared to MPPE-lacking vector regardless of the cell line used. Therefore, based on this limited analysis, MPPE does not display any obvious species tropism.

The present invention is also usable as a research tool to broaden the understanding of the positive posttranscriptional effect of the MMTV sequence spanning the 5'UTR-env junction region (MPPE). According to structural analyses it follows that the stem loop predicted to fold within MPPE (see below) functions as a nuclear export signal. Localization of this signal in the vicinity of the 5'end of mRNA (in the env or rem mRNA) allows the recruitment of factors to the newly synthesized transcript before other RNA-binding proteins (such as Rem/Crm1), thereby overruling the later export signals. As a result, the mRNA carrying MPPE is exported from the nucleus in a Rem/Crm1-independent pathway. By analogy to the recently described SSCR-mediated mRNA export, it is evident that the MPPE-containing mRNA recruits different mRNA-binding factors and is distributed in the cytoplasm differently to mRNAs lacking the MPPE signal (Fig. 11).

### 4.: Folding structure of the 5' end of the MMTV env mRNA

A computer-assisted prediction of a likely folding of the 5'terminal region derived from the MMTV env mRNA was performed and this folding was compared with the structure predicted for the 5'end of the MMTV gag/pol mRNA. In silico analysis, using the Mfold program (Zuker, Science 244 (1988), 48-52), revealed a long stem-loop coinciding with the 5'UTR-env boundary (Fig. 12; right panel). This stem-loop does not seem to be folded in the gag/pol mRNA (Fig. 12; left panel). It is evident that the identified stem-loop, which resembles the secondary structure of CTEs, is responsible for the ability to functionally replace posttranscriptional activation directed by Rev and RRE in the context of a HIV-1 Gag expression plasmid.

This can be analysed in detail by deletion mapping experiments wherein the minimal region necessary for Rev-independent expression of HIV-1 Gag can be easily demonstrated. Deletions in the 5' and 3' ends of the MPPE element in the p3-MPPE construct are made and the resulting constructs are analyzed for Gag expression by Western immunoblotting and using a commercial HIV-1 p24 antigen capture assay (Cell Biolabs, Inc.). To unequivocally demonstrate that the presence of MMPE facilitates the cytoplasmic accumulation of the HIV-1 gag mRNA, a Nothern blot analysis (using a HIV-1 gag-specific RNA probe) and a real time RT-PCR (HIV-1 gag-specific primers) for quantification of cytoplasmic and nuclear preparations are performed. Sub-cellular fractionation are controlled by a real time RT-PCR using primers homologous to the intron 6 of the unprocessed pre-mRNA of the glyceraldehyde-3-phosphatase dehydrogenase (pre-GAPDH mRNA) (Hohenadl et al., J. Gen. Virol. (2011)). Co-transfection of an GFP expression construct (pCMVeGFP) are used to monitor transfection efficiencies (Mullner et al., 2008).

## Claims

1. Use of the 5' terminal region of the env mRNA of Mouse Mammary Tumour Virus (MMTV) for enhancing nucleo-cytoplasmic translocation of a heterologous gene expressed in a host cell.

2. Use according to claim 1, wherein the 5' terminal region of the env mRNA of MMTV comprises nucleotides 1470 to 1608 and 6683 to 6871 of the MMTV reference strain BR6 (EMBL: M15122).

3. Use according to claim 1 or 2, wherein the heterologous gene is a viral gene, preferably a retroviral gene, especially a gene from the human immunodeficiency virus (HIV).

4. Use according to any one of claims 1 to 3, wherein the heterologous gene is a vaccination antigen.

5. Use according to any one of claims 1 to 4, wherein the heterologous gene is part of a DNA vaccine or part of a gene therapy vector.

6. Use according to any one of claims 1 to 5, wherein the host cell is a eukaryotic production cell line, preferably a CHO cell, a BHK cell, a Vero cell or a HEK-293 cell, especially HEK-293T cell; a cell of a non-human animal, preferably a fish, a bird or a mammal, especially a cat, a dog, a mouse, a hamster, a rat, a cow, a horse, a guinea pig, a goat, a rabbit, a chimpanzee, a macaque or a sheep ; or a human cell.

7. Use according to any one of claims 1 to 6, wherein the 5' terminal region of the env mRNA of MMTV and the heterologous gene are encoded on an expression vector, especially an expression plasmid or a viral expression vector.

8. Expression vector for a gene in a host cell for which the gene is heterologous, comprising the heterologous gene and elements enabling transcription and, optionally, translation of the gene in the host cell, **characterised in that** the vector further comprises the 5' terminal region of the env mRNA of MMTV.

9. Expression vector according to claim 8, further comprising one or more additional nucleus export elements, preferably selected from Rem responsive element (RmRE), constitutive transport element (CTE), Rev responsive element (RRE) or RNA transport element (RTE).

10. Expression vector according to claim 8 or 9, wherein the heterologous gene is a viral gene, preferably a retroviral gene, especially a HIV gene, an intronless gene or a spliced cDNA.

11. Expression vector according to any one of claims 8 to 10, further comprising a promoter derived from retroviruses, especially the Rous sarcoma virus (RSV) promoter, the spleen focus forming virus (SFFV) promoter), other viruses such as the cytomegalovirus (CMV) promoter, the herpes simplex virus thymidine kinase promoter(HSV TK),the simian virus 40 (SV40) promoter), promoters derived from eukaryotic organisms especially the elongation factor 1a (EF1a) promoter, the phosphoglycerate kinase 1 (PGK1) promoter and tissue-specific promoters, preferably *haematopoeitic-lineage promoters,* especially human ankyrin (ANK-1) promoter.

12. Method for expressing a heterologous gene in a host cell comprising introducing an expression vector according to any one of claims 8 to 11 into a host cell and inducing expression of the heterologous gene encoded by the expression vector.

13. Isolated nucleic acid comprising a gene expressible in a host cell, elements enabling transcription and, optionally, translation of the gene in the host cell, **characterised in that** the vector further comprises the posttranscriptional regulation of gene expression element of 5' terminal region of the env mRNA of MMTV.


SEQUENCE LISTING

<110>  VETERINÄRMEDIZINISCHE UNIVERSITÄT WIEN

<120>  Posttranscriptional Element

<130>  R 59637

<160>  10

<170>  PatentIn version 3.5

<210>  1
<211>  10125
<212>  DNA
<213>  Murine adenovirus

<400>  1
tgtgtgtgtg tgtgtgtgta cacgcttggg gggggagcgg ctgcgttctc ctggtcaagt      60

cagggggtgg ggtgcgggtg gggtggggat gccccgggct ctgggagggg ccgcagttcc     120

gtcagtgccg cgcctgcagc agaaatggtt gaactcccga gagtgtccta cacttagggg     180

agaagcagcc aaggggttgt ttcccaccaa ggacgacccg tctgcgcaca aacggatgag     240

cccatcagac aaagacatac tcattctctg ctgcaaactt ggcatagctc tgctttgcct     300

ggggctattg ggggaagttg cggttcgtgc tcgcagggct ctcacccttg actcttttaa     360

tagctcttct gtgcaagatt acaatctaaa cgattcggag aactcgacct tcctcctgag     420

gcaaggacca cagccaactt cctcttacaa gccgcatcaa ccttgtcctt cagaaataga     480

aataagaatg cttgctaaaa attatatttt taccaataag accaatccaa taggtagatt     540

attagttact atgttaagaa atgaatcatt accttttagt actattttta ctcaaattca     600

aaggttagaa atgggaatag aaaatagaaa gagacactca acctcagttg aagaacaggt     660

gcagggacta agggcctcag gcctagaagt aaaaagggga aagaggagtg cgcttgtcaa     720

aataggagac aggtggtggc aaccagggac ttatagggga ccttacatct acagaccaac     780

agatgccccc ttaccgtata caggaagata tgacctaaat tttgataggt gggtcacagt     840

caatggctat aaagtgttat acaggtccct ctcctttcgt gaaaggctcg ccagggctag     900

acctccttgg tgtatgttga ctcaggaaga gaaaaacgac atgaaacaac aggtacatga     960

ttatatttat ttgggaacag gaatgagcag catttgggga aagatttttc ataccaagga    1020

gaggacagtg gctgcactaa tagagcacta ttctgcaaag acttatggca tgagttatta    1080

tgattagcct ttattagccc aatcttgtgg ttcccaaggt ttaagtaagt tcatggtcac    1140

agactgttct taaaacaagg atgtgagaca agtggtttcc tgacttggct tggtatcaaa    1200

tgttttgatc taagctctaa atgctctaac ctcctatgtt cttttggatt ctatccaagt    1260

tttatgtaaa tgcttatgta aaccatgata taaaagagtg ctaaattttt gagtaaactt    1320

gcaacagtcc taacattcac ctctcgtgtg tttgtgtctg ttcgccatcc cgtctccgct    1380

cgtcacttat ccttcacttt ccagagggtc cccccgcaga ccccggtgac cctcaggtcg    1440

gccgactgcg gcagctggcg cccgaacagg gaccctcgga taagtgaccc ttgtctctat    1500

ttctactatt ttgtgttcgt cttgttttgt ctctatctta tctggctatt atcacaagag    1560

cggaacggac tcaccacagg gaactgcagt ctcgcctaca gagaagaggt aggttacggt 1620
gagccattgg aaatgggggt ctcgggatca aaagggcaga aactctttgt ttctgttcta 1680
caaagactcc tctcagagag gggtcttcat gtgaaagaga gcagtgcaat agagttttat 1740
cagttcctaa taaaggtctc tccttggttt cccgaagaag gaggattaaa tttacaagat 1800
tggaaaaggg tgggaagaga gatgaagagg tacgcagcag aacatgggac ggatagtata 1860
ccaaagcagg cttaccccat ttggcttcag ttgagagaga tactgacaga gcaatcagac 1920
ttggttttgt tatctgcaga agccaagtcc gtgactgaag aggaattaga ggaaggttta 1980
accggactac tatcgacaag ttcacaggaa aaaacttatg ggactagggg aacagcatat 2040
gcagaaatag atacagaggt agacaagctg tctgaacata tttatgatga accatatgaa 2100
gaaaaggaga aggcagataa aaatgaggaa aaggaccatg ttagaaaaat aaagaaggta 2160
gtacaaagaa aagaaaatag tgagggtaag agaaaagaga aggattcaaa ggccttttta 2220
gccacagatt ggaacgatga tgacctgtcc cctgaggatt gggacgattt agaggaacaa 2280
gcggcacatt atcatgatga tgatgagcta atccttccag taaaaaggaa ggtggttaag 2340
aagaaacctc aggcactcag aaggaaaccc ctgcctccgg tgggttttgc aggagcgatg 2400
gcagaggcca gggaaaaagg agatttgact tttacgtttc ctgtagtttt tatgggagag 2460
agtgatgaag atgacacgcc tgtttgggaa ccgctgccat tgaaaacctt aaaggaattg 2520
caatcggcag ttaggaccat gggaccatct gctccctaca ccctgcaggt ggtagacatg 2580
gtggctagtc aatggctcac cccgagtgac tggcaccaaa cagccagagc taccttgtct 2640
cctggagact atgttttatg gagaactgaa tatgaagaga aaagtaaaga aatggtacaa 2700
aaggctgcag gcaagcgaaa gggcaaggtc tctcttgata tgttactggg gactggccaa 2760
ttcctgtccc cttcttctca gataaaattg tctaaggatg tcttaaaaga tgtcaccaca 2820
aatgctgtgt tagcatggag ggccattccg cctcctggag ttaaaaagac tgtattagca 2880
ggattaaaac agggaaatga agagtcttat gagactttca tttcaaggct cgaggaagct 2940
gtttaccgaa tgatgcccag aggggaaggg tcggatatat tgatcaaaca attggcgtgg 3000
gagaatgcaa attcattgtg tcaggatctc atccgcccaa tacgtaaaac aggaactata 3060
caggattata ttcgtgcttg tctggacgct tctcccgcag ttgttcaggg tatggcatat 3120
gcagcagcca tgagaggaca aaagtattct acctttgtaa agcaaacata tggtggggga 3180
aaaggaggtc aaggagcaga agggccagtt tgtttttcct gtggtaagac aggacacatc 3240
agaaaagact gtaaggatga aaagggctca aaaagggccc ctcctgggct ctgccccaga 3300
tgtaagaaag gctatcactg gaagagtgag tgtaaatcta aatttgacaa agatgggaat 3360
ccacttcctc ccttggaaac taatgctgaa aattcaaaaa acttgtaaag gggcagtccc 3420
ctagccccgc tcaaaagggg gatggagtta agggctcagg attaaatcct gaagccccac 3480
ctttcacaat acatgatttg cctcgaggca cccctggaag tgcaggttta gacctgtcat 3540
cacagaagga tttgatcctc tctctagaag atggagtatc attggtaccc accttagtga 3600

aaggcaccct ccctgaaggg actactggac taataatagg tagaagttct aattataaaa 3660
agggacttga ggttttacca ggagtcattg actccgattt ccaaggagaa atcaaggtta 3720
tggttaaggc cgcaaaaaat gcggtcatca ttcacaaagg agaaagaata gcacaactgc 3780
tgttgctgcc atatttaaaa ttacccaatc ctgtaatcaa ggaagaacga ggctcagaag 3840
gcttcggatc aacaagtcat gtgcattggg tgcaggaaat aagtgactcc agacccatgc 3900
ttcacattta cttaaatgga agaagattcc tcggtctctt ggataccggg gcagataaaa 3960
cttgcatagc aggcagagac tggccagcta attggcccat tcaccaaact gagagttctc 4020
ttcaaggttt aggcatggcc tgtggggtgg cgcgtagtag tcagccactc cgttggcaac 4080
atgaggataa atcaggaatt atacatcctt ttgtgatccc tacactgcct ttcaccttat 4140
ggggaagaga tattatgaaa gatataaagg tcagattgat gacagactca ccagatgatt 4200
cacaggattt atgatagggg ccattgagag caatctcttt gcagaccaaa tatcttggaa 4260
gtcagaccag cctgtatggc ttaatcaatg gccccttaaa caagaaaagt tacaggcttt 4320
acaacagtta gtgacagaac aattacaact gggccactta gaagagagca atagcccttg 4380
gaatacgcct gtttttgtca ttaaaaagaa gtcaggaaaa tggagactgt tacaagacct 4440
acgtgcagtt aatgctacaa tgcacgatat gggagcatta caacccggct tgccgtcccc 4500
tgtagcagtc cctaaaggat gggaaataat cataatagat ctacaagatt gcttttttaa 4560
tataaaactg catcctgaag attgtaaaag atttgctttt agtgtgccct cccctaattt 4620
taagagacct tatcaaagat tccaatggaa agttttgccc cagggtatga aaaatagccc 4680
tactttatgt caaaaatttg tggacaaagc tatattgact gtaagggata aataccaaga 4740
ctcatatatt gtgcattaca tggatgacat tcttttggca cacccatcaa gatccattgt 4800
cgatgaaata cttacttcca tgatacaggc ccttaacaaa catggccttg tagtatccac 4860
agagaagatt caaaaatatg ataatctcaa atatttggga actcatatac agggtgattc 4920
agtgtcttat caaaaattac agattaggac agataaatta agaaccttaa atgatttcca 4980
aaagctatta ggaaatatta attggatacg tcctttctta aaattaacta cgggagagtt 5040
aaaacctctc tttgaaattc ttaatggaga ttctaatccg atctcaacaa gaaaacttac 5100
tcctgaggca tgcaaagctc ttcaattaat gaatgagaga ctatctaccg ctcgggtaaa 5160
gaggctagat ttatcacagc cttggtctct atgtatatta aagactgaat atacccccac 5220
agcatgcctc tggcaggatg gagttgtaga atggatacat ttgcctcata tttcaccaaa 5280
ggtgattact ccttatgata tcttttgtac acaacttatt attaagggcc gacaccgctc 5340
taaagaatta tttagtaaag accctgatta tattgttgtg ccctacacca aagttcaatt 5400
tgatctccta ttacaagaaa aggaagattg gcctatttct ttattagggt tcttgggaga 5460
ggttcatttc catcttccaa aagacccttt gcttacattt accctacaaa ctgccattat 5520
ttttcctcac atgacctcta ccacaccact agagaaagga attgtgattt ttacagacgg 5580
gtcagcaaat ggccgttcgg taacatatat acaaggaagg gagcctataa ttaaagaaaa 5640
tacacaaaac acagcccaac aggctgaaat tgtggcagtc attacagcct ttgaggaagt 5700

gagtcaaccc tttaatttgt atactgattc taaatatgtg acagggttgt ttcccgaaat 5760
cgaaactgca actttgtcac ccagaacaaa aatttacaca gaactgaaac atttacaaag 5820
gttaatccac aagagacaag aaaaatttta cattggtcat atcagaggac acactggact 5880
tcccggtcct ttggcacagg gaaatgccta tgcagattct ttaacaagaa ttctgaccgc 5940
tttagagtca gctcaagaaa gccacgcact acatcatcaa aatgccgcgg cgcttaggtt 6000
tcagtttcac atcactcgtg aacaagcgcg agaaatagta aaattatgtc ccaattgccc 6060
cgactggggg cacgcgccgc aattaggggt aaaccccagg ggccttaagc cccgagttct 6120
atggcaaatg gatgttactc atgtttcaga atttggaaaa ttaaaatatg tacatgtgac 6180
agtggatact tattctcatt ttactttcgc taccgcccga acgggcgaag caaccaagga 6240
tgtgttacaa cacttggctc aaagctttgc atacatgggc attcctcaaa aaataaaaac 6300
agataatgcc cctgcatatg tgtctcgttc aatacaagaa tttctggcca gatggaaaat 6360
atctcacgtc acggggatcc cctacaatcc ccaaggacag gccattgttg aacgaacgca 6420
ccaaaatata aaggcacagc ttaataaact tcaaaaggct ggaaaatact atacacccca 6480
tcatctgttg gcacacgctc tttttgtgct gaatcatgta aatatggaca atcaaggcca 6540
tacagcggcc gaaagacatt ggggtccaat ctcagccgat ccaaaaccta tggtcatgtg 6600
gaaagacctt ctcacagggt cctggaaagg acccgatgtc ctaataacag ccggacgagg 6660
ctatgcttgt gtttttccac aggatgccga aacaccaatc tgggtccccg accgattcat 6720
ccgacctttt actgagcgga aagaagcaac gcccacacct ggcactgcgg agaaaacgcc 6780
gccgcgagat gagaaagatc aacaggaaag tcccaagaat gaatctagtc cccatcaaag 6840
agaagacggc ttggcaacat ctgcaggcgt tgatctccga agcggaggag gtccttaaaa 6900
cctcacaaac tccccaaacc tctttgacct tatttcttgc tttgttgtct gtcctcggcc 6960
ccccgcctgt gacaggggag agttattggg cctacctacc taaaccacct attctccatc 7020
ccgtgggatg gggaagtaca gaccccatta gagttctgac aaatcaaacc atgtatttgg 7080
gtggttcgcc tgactttcat gggttcagaa atatgtctgg taatgtacat tttgagggga 7140
agtctgatac gctccccatt tgcctttcct tctccttttc tacccccacg ggctgctttc 7200
aagtagacaa gcaagtattt ctttctgata cacccacggt tgataataat aaacctgggg 7260
gaaagggtga taaaaggcgt atgtgggaac tttggttgac taccttgggg aactcagggg 7320
ccaatacaaa actggtccct ataaaaaaga agttgccccc caaatatcct cactgccaga 7380
tcgcctttaa gaaggacgcc ttctgggagg gagacgagtc tgctcctcca cggtggttgc 7440
cttgcgcctt ccctgaccag ggggtgagtt tttctccaaa aggggccctt gggttacttt 7500
gggatttctc ccttccctcg cctagtgtag atcagtcaga tcagattaaa agcaaaaaga 7560
atctatttgg aaattatact ccccctgtca ataaagaggt tcatcgatgg tatgaagcag 7620
gatgggtaga acctacttgg ttctgggaaa attctcctaa ggatcccaat gatagagatt 7680
tcactgcact agtcccccat acagaattgt ttcgcttagt cgcagcctca agacatctta 7740

ttctcaaaag gccaggattt caagaacatg agatgattcc tacatctgcc tgtgttactt 7800
acccttatgc catattatta ggattacctc agttaataga tatagagaaa agaggatcta 7860
cttttcatat ttcctgttct tcttgtagat tgactaattg tttagactct tctgcctacg 7920
actatgcagc gatcatagtc aagaggccgc catatgtgct gctacctgta gatattggtg 7980
atgaaccatg gtttgatgat tctgccattc aaacctttag gtatgccaca gatttaattc 8040
gagctaagcg attcgtcgca gccattatcc tgggcatatc tgctttgatt gctattatta 8100
cttcctttgc tgtagctact actgccttag ttaaggagat gcaaactgct acgtttgtta 8160
ataatcttca taggaatgtt acattagcct tatctgaaca acggataata gatttaaaat 8220
tagaagctag acttaatgct ttagaagaag tagttttaga gttgggacaa gatgtggcca 8280
atttaaagac cagaatgtcc actaggtgtc atgcaaatta tgactttatc tgcgttacac 8340
ctttacccta taatgctact gagaactggg aaagaaccag ggctcattta ttgggcattt 8400
ggaatgataa tgagatttca tataacatac aggagttaac caacctgatt agtgatatga 8460
gcaaacaaca tattgatgca gtggacctta gtggcttggc tcagtctttt gccaatggag 8520
tgaaggcttt aaatccatta gattggacac aatatttcat ttttataggt gttggagccc 8580
tgcttttagt cattgtactt atgattttcc ccattgtttt ccagtgcctt gcgaagagcc 8640
ttgaccaagt gcagtcagat cttaacgtgc ttcttttaaa aaagaaaaaa gggggaaatg 8700
ccgcgcctgc agcagaaatg gttgaactcc cgagagtgtc ctacacttag gggagaagca 8760
gccaaggggt tgtttcccac caaggacgac ccgtctgcgc acaaacggat gagcccatca 8820
gacaaagaca tactcattct ctgctgcaaa cttggcatag ctctgctttg cctggggcta 8880
ttgggggaag ttgcggttcg tgctcgcagg gctctcaccc ttgactcttt taatagctct 8940
tctgtgcaag attacaatct aaacgattcg gagaactcga ccttcctcct gaggcaagga 9000
ccacagccaa cttcctctta caagccgcat caaccttgtc cttcagaaat agaaataaga 9060
atgcttgcta aaaattatat ttttaccaat aagaccaatc caataggtag attattagtt 9120
actatgttaa gaaatgaatc attacctttt agtactattt ttactcaaat tcaaaggtta 9180
gaaatgggaa tagaaaatag aaagagacac tcaacctcag ttgaagaaca ggtgcaggga 9240
ctaagggcct caggcctaga agtaaaaagg ggaaagagga gtgcgcttgt caaaatagga 9300
gacaggtggt ggcaaccagg gacttatagg ggaccttaca tctacagacc aacagatgcc 9360
cccttaccgt atacaggaag atatgaccta aattttgata ggtgggtcac agtcaatggc 9420
tataaagtgt tatacaggtc cctctccttt cgtgaaaggc tcgccagggc tagacctcct 9480
tggtgtatgt tgactcagga agagaaaaac gacatgaaac aacaggtaca tgattatatt 9540
tatttgggaa caggaatgag cagcatttgg ggaaagattt ttcataccaa ggagaggaca 9600
gtggctgcac taatagagca ctattctgca aagacttatg gcatgagtta ttatgattag 9660
cctttattag cccaatcttg tggttcccaa ggtttaagta agttcatggt cacagactgt 9720
tcttaaaaca aggatgtgag acaagtggtt tcctgacttg gcttggtatc aaatgttttg 9780
atctaagctc taaatgctct aacctcctat gttcttttgg attctatcca agttttatgt 9840

aaatgcttat gtaaaccatg atataaaaga gtgctaaatt tttgagtaaa cttgcaacag     9900

tcctaacatt cacctctcgt gtgtttgtgt ctgttcgcca tcccgtctcc gctcgtcact     9960

tatccttcac tttccagagg gtccccccgc agaccccggt gaccctcagg tcggccgact     10020

gcggcacgtc agctcgagca gaaccacccg tgagttaggt cgagcagagc caaagccccc     10080

ggtgcttcgt cgcgggttcg ctcgctagct atctggatca ctccc                    10125


<210>  2
<211>  328
<212>  DNA
<213>  Mouse mammary tumor virus

<400>  2
ggaccctcgg ataagtgacc cttgtctcta tttctactat tttgtgttcg tcttgttttg       60

tctctatctt atctggctat tatcacaaga gcggaacgga ctcaccacag ggaactgcag      120

tctcgcctac agagaagagg atgccgaaac accaatctgg gtccccgacc gattcatccg      180

accttttact gagcggaaag aagcaacgcc cacacctggc actgcggaga aaacgccgcc      240

gcgagatgag aaagatcaac aggaaagtcc caagaatgaa tctagtcccc atcaaagaga      300

agacggcttg gcaacatctg caggcgtt                                         328


<210>  3
<211>  34
<212>  DNA
<213>  Mouse mammary tumor virus

<400>  3
aaaaaactcg agtcagcact cttttatatt atgg                                   34


<210>  4
<211>  32
<212>  DNA
<213>  Mouse mammary tumor virus

<400>  4
aaaaaaaagc ttgcaacagt cctaacattc ac                                     32


<210>  5
<211>  33
<212>  DNA
<213>  Mouse mammary tumor virus

<400>  5
aaaaaatcta gactaagtgt aggacactct cgg                                    33


<210>  6
<211>  36
<212>  DNA
<213>  Mouse mammary tumor virus

<400>  6
aaaaaaaagc ttattcacct ctcgtgtgtt tgtgtc                                 36


<210>  7
<211>  32

<212>  DNA
<213>  Mouse mammary tumor virus

<400>  7
aaaaaaaagc ttctcttctc tgtaggcgag ac                                    32


<210>  8
<211>  18
<212>  DNA
<213>  Mouse mammary tumor virus

<400>  8
tacgggtgat ggggaaag                                                    18


<210>  9
<211>  20
<212>  DNA
<213>  Mouse mammary tumor virus

<400>  9
tcctcccctg ggaagacttg                                                  20


<210>  10
<211>  236
<212>  RNA
<213>  Mouse mammary tumor virus

<400>  10
gacccucgga uaagugaccc uugucucuau uucuacuauu uuguguucgu cuuguuuugu      60

cucuaucuua ucuggcuauu aucacaagag cggaacggac ucaccacagg gaacugcagu     120

cucgccuaca gagaagagga ugccgaaaca ccaaucuggg uccccgaccg auucauccga     180

ccuuuuacug agcggaaaga agcaacgccc acaccuggca cugcggagaa aacgcc         236